# EUROPEAN PATENT SPECIFICATION

(11) **EP 2 123 662 B1**
(45) Date of publication and mention of the grant of the patent: **04.12.2013**
(21) Application number: 08704137.2
(22) Date of filing: 30.01.2008
(51) Int. Cl.: C07H 3/04, A61K 31/7016, A61P 31/00, A61P 35/00, A61P 37/04, A61P 43/00

(54) **TREHALOSE COMPOUND, PROCESS FOR PRODUCTION OF THE COMPOUND, AND IMMUNO-STIMULATIVE AGENT COMPRISING THE COMPOUND**
TREHALOSEVERBINDUNG, VERFAHREN ZUR HERSTELLUNG DER VERBINDUNG UND DIE VERBINDUNG ENTHALTENDES, DAS IMMUNSYSTEM STIMULIERENDES MITTEL
COMPOSÉ TRÉHALOSE, PROCÉDÉ DE FABRICATION DU COMPOSÉ ET AGENT IMMUNOSTIMULANT COMPRENANT LE COMPOSÉ

(30) Priority: 31.01.2007 JP 2007021227
(43) Date of publication of application: 25.11.2009
(73) Proprietor: GLYTECH, INC., Shimogyo-ku, Kyoto-shi, Kyoto 600-8813 (JP)
(72) Inventor: NISHIZAWA, Mugio, Tokushima-shi Tokushima 770-0872 (JP); IMAGAWA, Hiroshi, Tokushima-shi Tokushima 770-0866 (JP); YAMAMOTO, Hirofumi, Tokushima-shi Tokushima 770-0944 (JP); SAKURAI, Jun, Tokushima-shi Tokushima 771-4263 (JP); ODA, Masataka, Tokushima-shi Tokushima 770-0944 (JP)
(74) Representative: Müller-Boré & Partner Patentanwälte
(86) International application number: PCT/JP2008/051355
(87) International publication number: WO 2008/093700

(56) References cited:
- WO-A1-2007/111214
- JP-A- 03 251 537
- JP-A- 10 072 478
- JP-A- 61 130 298
- JP-A- 2005 281 298
- LASZLO A ET AL: "Screening of synthetic trehalose 6,6'-diesters and trehalose 6-monoesters as potential immunoreactants for the serodiagnosis of tuberculosis" RESEARCH IN MICROBIOLOGY, ELSEVIER, AMSTERDAM, NL, vol. 145, no. 7, 1 January 1994 (1994-01-01), pages 563-572, XP023925004 ISSN: 0923-2508 [retrieved on 1994-01-01]
- NUMATA FUMIO ET AL.: 'Lethal and adjuvant activities of cord factor (trehalose 6,6'-dimycolate) and synthetic analogs in mice' CHEMICAL & PHARMACEUTICAL BULLETIN vol. 33, no. 10, 1985, pages 4544 - 4555, XP008111645

## Description

### TECHNICAL FIELD

The present invention relates to a trehalose compound, a process for producing the compound, and an immunostimulating agent comprising the compound.

### BACKGROUND ART

To date, anti-carcinogenic agents such as paclitaxel, mitomycin C, futraful, irinotecan, and cisplatin have been clinically employed around the world as leading anti-carcinogenic agents. An anti-carcinogenic agent is a cytotoxic substance, which is a compound that is highly selective for the preferential killing of cancer cells with respect to normal cells.

However, since the selectivity of these anti-carcinogenic agents is not displayed in immunocytes undergoing intense cell division, problematic side effects related to anti-carcinogenic agents, in which the physical condition of the patient is compromised by the remarkable damage incurred by the patient's immunocytes, have not yet been resolved. Recently, in light of such problems, several types of cancer therapies have been tested without the employment of anti-carcinogenic agents, via the strengthening of the immunity of the patients themselves.

Trehalose 6,6'-dimycolate, in which two mycolic acid molecules are ester-linked at the 6,6'-position of trehalose (TDM, the compound represented by formula (A) below), is known to demonstrate an anti-carcinogenic activity via immunological activation (Non-Patent Documents 1 and 2).

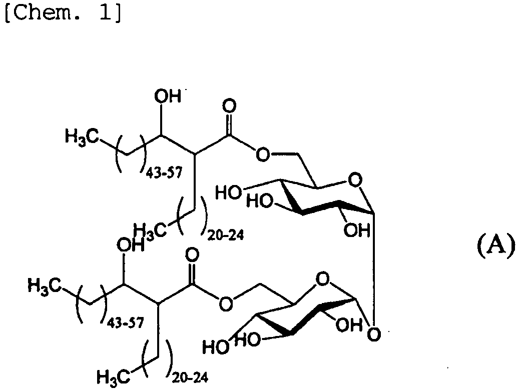

However, since TDM demonstrates highly lethal activity in mice, the practical applications thereof cannot be attained without first resolving the problems regarding the intrinsic toxicity thereof.

The present inventors succeeded in the synthesis of diastereoisomers of trehalose 6,6'-dicorynomycolate, in which two corynomycolic acid molecules are ester-linked at the 6,6'-position of trehalose (TDCM, the compound represented by formula (B) below), and found that each of the TDCMs demonstrates an anti-tumor activity through an immunostimulatory action (Non-Patent Document 3).

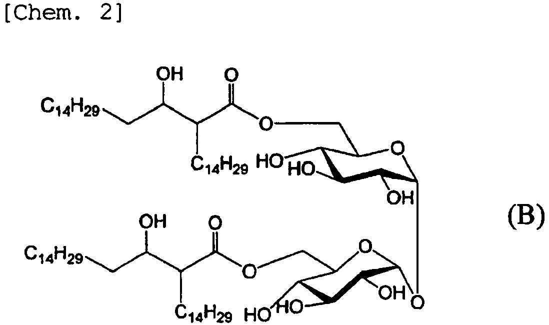

However, while TDCM demonstrates an immunostimulatory action, the effects thereof are insufficient. Moreover, since asymmetric synthesis occurs during the production of TDCM, a substantial amount of labor is required, and thus it is difficult to supply TDCM in large quantities.
JP 61 130298 discloses α,α-trehalose fatty acide diamide derivatives for use as carcinostatic agents.

### Non-Patent Documents

Non-Patent Document 1: Chem. Pharm. Bull., 33, 4544 (1985)
Non-Patent Document 2: J. Carbohydr. Chem., 5, 127 (1986)
Non-Patent Document 3: Synlett, 452 (1996)

### DISCLOSURE OF THE INVENTION

### Problem to be Solved by the Invention

An object of the present invention is to provide a novel trehalose compound that has an excellent immunostimulating effect and that can be supplied in a large quantity on an industrial scale, and a method for producing the trehalose compound.

### Means for Solving the Problem

The present inventors conducted extensive research in order to achieve the aforementioned object. As a result, the inventors found that a trehalose compound having greatly improved immunostimulatory activity can be obtained by making the ester bond of TDCM into an amide bond, and replacing the hydroxyl group at β-position, which is a characteristic of the corynomycolic acid site of TDCM, with a hydrogen atom. The present invention was completed based on this finding.

The present invention provides the trehalose compounds, methods for producing the same, and immunostimulating agents comprising the trehalose compound described in Items 1 to 5 below.
Item 1. A trehalose compound represented by general formula (1):

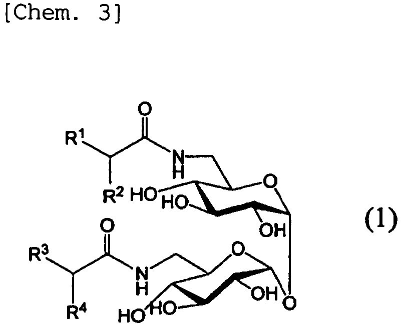

wherein R¹, R², R³ and R⁴ may be the same or different, and independently represent a C₁₀₋₁₆alkyl group.
Item 2. A process for producing a trehalose compound represented by general formula (1):

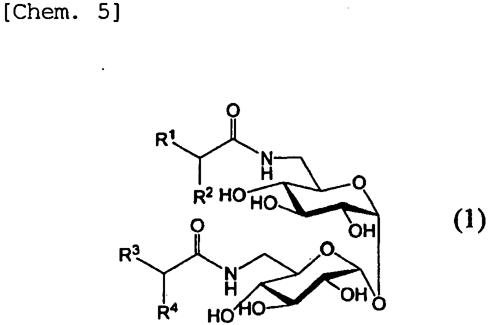

wherein R¹, R², R³ and R⁴ may be the same or different, and independently represent a C₁₀₋₁₆alkyl group;
the process comprising the step of removing a protective group for a hydroxyl group from a trehalose compound represented by general formula (5):

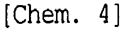

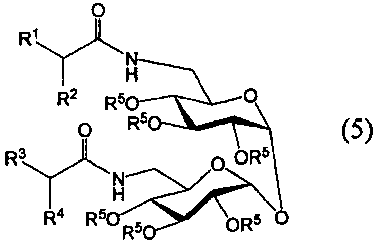

wherein R¹, R², R³ and R⁴ are the same as above, and R⁵ is a protective group for a hydroxyl group.
Item 3. A process for producing a trehalose compound represented by general formula (1):

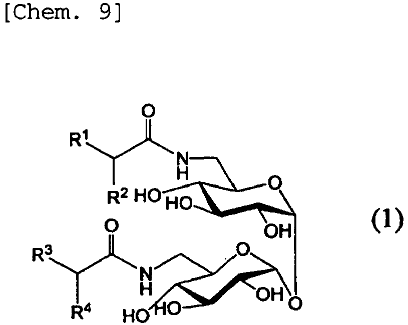

wherein R¹, R², R³ and R⁴ may be the same or different, and independently represent a C₁₀₋₁₆ alkyl group;
the process comprising the steps of:
simultaneously or sequentially reacting a trehalose compound represented by general formula (2):

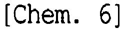

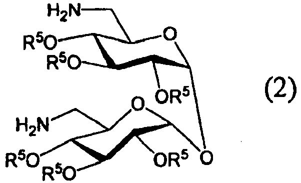

wherein R⁵ represents a protective group for hydroxyl group, with a carbonyl compound represented by general formula (3) and a carbonyl compound represented by general formula (4):

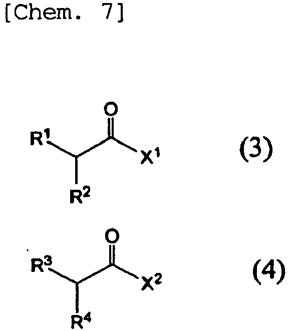

wherein R¹, R², R³ and R⁴ may be the same or different, and independently represent a C₁₀₋₁₆ alkyl group, and X¹ and X² may be the same or different, and independently represent a hydroxyl group or a halogen atom; and
removing a protective group for a hydroxyl group from the resulting trehalose compound represented by general formula (5):

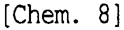

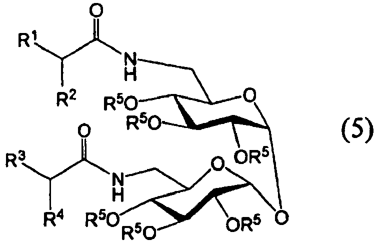

wherein R¹, R², R³, R⁴ and R⁵ are the same as above.
Item 4. An immunostimulating agent comprising the trehalose compound defined by Item 1 as an active ingredient.
Item 5. A macrophage immunostimulating agent comprising the trehalose compound defined by Item 1 as an active ingredient.
Item 6. An immunostimulating agent for stimulating activity of neutrophil comprising the trehalose compound defined by Item 1 as an active ingredient.
Item 7. An immunostimulating agent for stimulating the phagocytosis of phagocytes comprising the trehalose compound defined by Item 1 as an active ingredient.
Item 8. An anti-microbism agent comprising the trehalose compound defined by Item 1 as an active ingredient.

In general formula (1), R¹, R², R³ and R⁴ are independently a straight- or branched-chain alkyl group having 10 to 16 carbon atoms. Preferable examples thereof include *n*-decyl, *n*-undecyl, *n*-dodecyl, *n*-tridecyl, *n*-tetradecyl, *n*-pentadecyl, *n-*hexadecyl and like straight-chain alkyl groups having 10 to 16 carbon atoms. Among these, straight-chain alkyl groups having 11 to 15 carbon atoms are particularly preferable, and *n*-tridecyl, i.e., a straight-chain alkyl group having 13 carbon atoms, and *n-*tetradecyl, i.e., a straight-chain alkyl group having 14 carbon atoms, are most preferable.

The trehalose compound of the present invention represented by general formula (1) can be obtained by, for example, the amidation reaction shown as synthesis scheme 1 below.

### Synthesis scheme 1

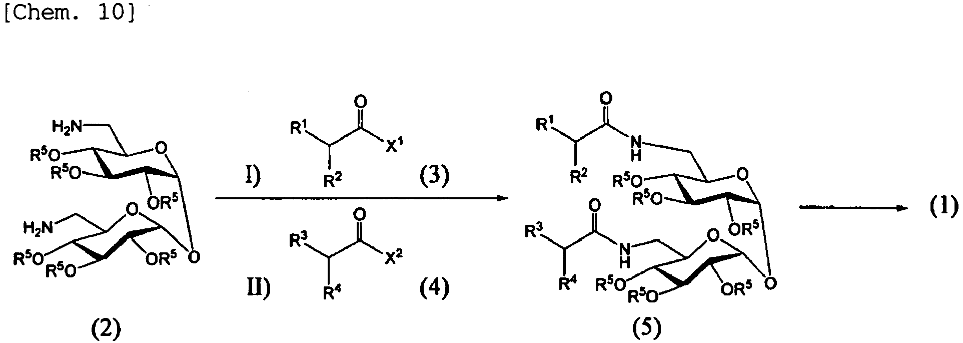

wherein R¹, R², R³, R⁴, R⁵, X¹ and X² are the same as above.
As shown in synthesis scheme 1, the trehalose compound (1) of the present invention can be obtained by simultaneously or sequentially reacting the trehalose compound represented by general formula (2) with the carbonyl compound represented by general formula (3) and the carbonyl compound represented by general formula (4), and removing the protective group for hydroxyl group from the resulting trehalose compound represented by general formula (5).

When carbonyl compound (3), wherein X¹ is a hydroxyl group, or carbonyl compound (4), wherein X² is a hydroxyl group is used, examples of employable reactions for forming trehalose compound (5) from trehalose compound (2) include various amidation reactions between carboxylic acid and amine, for example, a dehydrating method, a mixed acid anhydride method, an active esterification method, etc. It is preferable that these methods be conducted in an inert solvent using a condensing agent (including dehydrators).

Examples of condensing agents usable in the above amidation reaction include dehydrating agents such as hydrogen chloride, sulfuric acid, hydrochloric acid and like mineral acids; para toluene sulfonic acid, camphorsulfonic acid and like organic acids; boron fluoride etherate and like Lewis acids; phosphorus trichloride, phosphorus tribromide, phosphorus pentachloride, phosphorus oxychloride, thionyl chloride and like acid halide formation agents; ethyl chloroformate, methane sulfonyl chloride and like mixed acid anhydride formation agents; *N*,*N*'-dicyclohexylcarbodiimide (DCC), diisopropylcarbodiimide, 1-ethyl-3-(dimethylaminopropyl)carbodiimide and like carbodiimides; and other condensing agents such as *N,N*-carbonyldiimidazole, 2-ethoxy-*N*-ethoxycarbonyl-1,2-dihydroquinoline (EEDQ), triphenyl phosphine-carbon tetrachloride (complex), etc. These condensing agents may be used singly or in combination.

Various known solvents can be used as the inert solvent as long as they do not adversely affect the amidation reaction. Examples of usable solvents include benzene, toluene, xylene and like aromatic hydrocarbons; chlorobenzene, dichlorobenzene and like halogenated aromatic hydrocarbons; *n*-hexane, cyclohexane, petroleum ether and like aliphatic hydrocarbons; dichloromethane, 1,2-dichloroethane, chloroform, carbon tetrachloride and like aliphatic halogenated hydrocarbons; diethyl ether, diisopropyl ether, dioxane, tetrahydrofuran, ethylene glycol dimethyl ether, ethylene glycol diethyl ether and like ethers; acetone, 2-butanone, methyl isobutyl ketone and like ketones; acetonitrile, propionitrile, benzonitrile and like nitriles; *N,N*-dimethyl formamide, hexamethylphosphoric triamide (HMPA) and like amides; dimethyl sulfoxide and like sulfoxides; or a mixture of these solvents.

In order to promote the amidation reaction, dimethyl formamide, dimethylamido pyridine, 4-pyrrolidinopyridine and like catalysts; and anhydrous magnesium sulfate, molecular sieves (4A, 5A) and like drying agents may be added to the reaction system. A Dean-Stark water separator, Soxhlet extractor, etc., may also be used in the amidation reaction.

There is no limitation to the amount of the compound supplied as a material for the amidation reaction, and the amount may be selected from a wide range. When trehalose compound (2) is reacted with carbonyl compound (3) first, and the resulting reaction product is then reacted with carbonyl compound (4), generally 0.5 to 1.8 moles, and preferably 0.8 to 1.2 moles of carbonyl compound (3) is added; and generally 0.5 to 1.8 moles, and preferably 0.8 to 1.2 moles of condensing agent is added per one mole of trehalose compound (2). Alternatively, per one mole of reaction product of trehalose compound (2) and carbonyl compound (3), generally 0.5 to 1.8 moles, and preferably 0.8 to 1.2 moles of carbonyl compound (4) is added; and generally 0.5 to 1.8 moles, and preferably 0.8 to 1.2 moles of condensing agent is added.

There is no limitation to the reaction temperature, which generally may be selected within the range from -10°C to a temperature not exceeding the boiling point of the solvent used. The reaction time depends on the type and amount of the material compound, the reaction temperature and like reaction conditions, but can be appropriately selected within the range of from 3 to 10 hours.

When carbonyl compound (3) wherein X¹ represents halogen atom, or carbonyl compound (4) wherein X² represents halogen atom is used, the reaction may be conducted in an appropriate solvent, and in the presence of a base, if necessary.

The inert solvents usable in the amidation reaction mentioned above can also be used in this reaction.

Examples of bases include sodium hydroxide, potassium hydroxide and like hydroxides of alkali metals; calcium hydroxide and like hydroxides of alkaline earth metals; sodium carbonate, potassium carbonate and like carbonates of alkali metals; sodium hydrogencarbonate, potassium hydrogencarbonate and like hydrogencarbonates of alkali metals; sodium acetate, potassium acetate and like acetates of alkali metals; calcium acetate and like acetates of alkaline earth metals; sodium hydride, potassium hydride and like hydrides of alkali metals; calcium hydride and like hydrides of alkaline earth metals; ammonium hydroxide, ammonium carbonate, ammonium acetate and like ammonium salts; and trimethylamine, triethylamine, *N,N*-dimethylaniline, pyridine, 4-(dimethylamino)pyridine, diazabicyclooctane (DABCO), diazabicyclononene (DBN), diazabicycloundecene (DBU) and like tertiary amines. These bases may be used singly or in combination.

As in the amidation reaction described above, the amount of the material compound used in this reaction is not limited, and may be suitably selected from a wide range. When trehalose compound (2) is reacted with carbonyl compound (3), and the resulting reaction product is subsequently reacted with carbonyl compound (4), generally 0.5 to 1.8 moles, and preferably 0.8 to 1.2 moles of carbonyl compound (3) is added; and generally 0.5 to 1.8 moles, and preferably 0.8 to 1.2 moles of base is added per one mole of trehalose compound (2). Alternatively, per one mole of reaction product of trehalose compound (2) and carbonyl compound (3), generally 0.5 to 1.8 moles, and preferably 0.8 to 1.2 moles carbonyl compound (4) is added; and generally 0.5 to 1.8 moles, and preferably 0.8 to 1.2 moles of base is added.

As in the amidation reaction described above, there is no limitation to the reaction temperature, which is generally selected within the range from -10°C to a temperature not exceeding the boiling point of the solvent used. As in the amidation reaction described above, the reaction time depends on the content of the material compound, the reaction temperature and the like; however, the reaction time can be appropriately selected within the range from 0.1 to 10 hours.

When trehalose compound (2) is reacted with carbonyl compound (3) or (4), trehalose compound (2) may be reacted with carbonyl compound (3) and carbonyl compound (4) simultaneously. However, unless carbonyl compound (3) is identical with carbonyl compound (4), it is preferable that carbonyl compound (3) be reacted first and carbonyl compound (4) be reacted subsequently, so as to selectively produce trehalose compound (5)

When a carbonyl compound wherein X¹ represents a hydroxyl group and carbonyl compound wherein X² represents a halogen atom are used; and when a carbonyl compound wherein X¹ represents a halogen and carbonyl compound wherein X² represents a hydroxyl group are used, the objective trehalose compound represented by general formula (5) can be obtained by suitably selecting the reaction conditions.

When carbonyl compound (3) is identical with carbonyl compound (4), trehalose compound (5) is produced according to synthesis scheme 2 shown below.

### Synthesis scheme 2

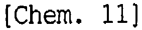

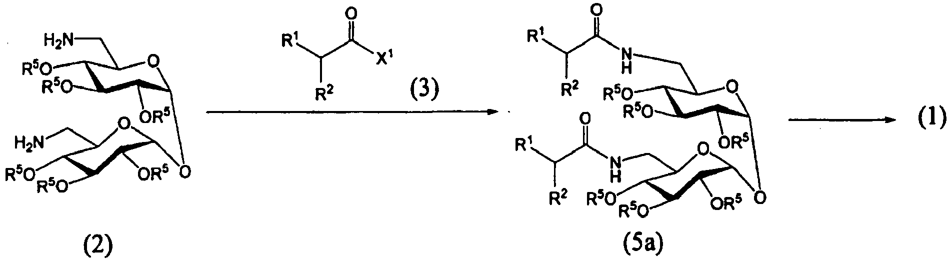

wherein R¹, R², R⁵ and X¹ are the same as above.
The reaction conditions for synthesis scheme 2 are the same as those in synthesis scheme 1, except that the amount of carbonyl compound (3) is increased. Specifically, per one mole of trehalose compound (2), generally 1.8 to 5 moles, and preferably 2 to 3 moles of carbonyl compound (3) is added; generally 1.8 to 5 moles, and preferably 2 to 4 moles of condensing agent is added; and generally 1.8 to 8 moles, and preferably 2 to 6 moles of base is added.

The resulting trehalose compound (5) or (5a) may be used in the subsequent reaction without isolating and purifying. However, it is preferable that the reagent used in the amidation reaction and the by-product formed in the reaction be removed.

R⁵ in the trehalose compounds (2), (5) and (5a) may be any known protective groups for hydroxyl group. For example, protective groups for hydroxyl group disclosed in Protecting Groups in Organic Chemistry (John Wiley & Sons, Inc., New York 1991, ISBN 0-471-62301-6) can be used. Specific examples thereof include benzyl, *p*-methoxybenzyl, biphenylmethyl and like arylalkyls; acetyl and like acyls; methoxycarbonyl, tert-butoxycarbonyl and like alkoxycarbonyls; and trimethylsilyl and like trialkylsilyls. Among these, benzyl is particularly preferable.

The protective group can be removed from the protected hydroxyl group in trehalose compound (5) or (5a) by, for example, selecting the deprotection method suitable for the protective group to be removed, as disclosed in the above-mentioned literature.

For example, when the protective group R⁵ is benzyl, a catalytic hydrogenation reaction can be employed. The catalytic hydrogenation reaction is conducted in the presence of a catalyst under a hydrogen atmosphere.

Various known catalysts can be used as long as they are usable in the catalytic hydrogenation reaction. Specific examples thereof include platinum oxide, platinum carbon, palladium hydroxide, palladium carbon, Raney nickel, etc.

The amount of the catalyst used is generally about 0.001 to 50 weight%, and preferably about 0.01 to 10 weight% relative to the weight of trehalose compound (5) or (5a).

There is no limitation to the pressure of hydrogen, which can be suitably selected from a wide range; the pressure is generally 0.8 to 100 atm, and preferably about 1 to 3 atm.

It is preferable that the reaction be conducted in a solvent. Examples of usable solvents include dichloromethane, 1,2-dichloroethane, chloroform, carbon tetrachloride and like aliphatic halogenated hydrocarbons; methanol, ethanol, isopropanol and like alcohols; methyl formate, methyl acetate, ethyl acetate and like esters; formic acid, acetic acid and like carboxylic acids; or mixtures of these solvents.

The reaction temperature of the present invention is generally 0 to 100°C, and preferably about 10 to 40°C. The reaction time depends on the weight of the material, the temperature, the type of the catalyst, etc., and can be selected based on the theoretical consumption amount of hydrogen such that the reaction can be completed.

All of the trehalose compound (2), carbonyl compound (3) and carbonyl compound (4) used as starting materials for the above reaction are commercially available, or can be produced by a known method.

For example, trehalose compound (2) can be produced based on the method disclosed in Bioorganic Medical Chemistry, 12, 6397 (2004).

Carbonyl compounds (3) and (4) can be obtained by, for example, referring to the method disclosed in Journal of American Chemical Society, 92, 1397 (1970) and combining it with known methods, if necessary.

The thus-obtained trehalose compound (1) can be easily isolated from the reaction mixture and purified by generally employed isolation and purification methods, such as column chromatography, recrystallization, etc.

As is clear from the results of the Experimental Examples below, the thus-obtained trehalose compound (1) of the present invention exhibits excellent immunostimulating activity. Therefore, the trehalose compound (1) of the present invention is useful as an active ingredient for a medical composition (immunostimulating agent) for treating cancer.

The present invention provides an immunostimulating agent that contains the trehalose compound (1) as an active ingredient (hereunder, the immunostimulating agent may be referred to as a "preparation").

The preparation of the present invention may consist of the trehalose compound (1), or may be a composition prepared into a form desirable for its usage by a known method and by adding appropriate carriers and/or additives.

There is no limitation to the form of the preparation of the present invention. Examples of such forms include tablets, powders, granules, pills, powder syrups, capsules (hard capsules and soft capsules) and like solid preparations; creams, ointments, gels and like paste-like or gelatinous preparations; solutions, suspensions, emulsions, syrups, elixirs and like liquid preparations; etc.

There is no limitation to the content of the trehalose compound (1) in the preparation of the present invention, as long as satisfactory immunostimulating activity can be achieved. The content of the trehalose compound (1) is generally 0.001 to 99 weight%, preferably 0.01 to 50 weight%, and more preferably 0.1 to 30 weight% per 100 weight% of the preparation.

There is no limitation to the content of the trehalose compound (1), and other components may be added in an amount within the range that does not hinder the preparation from exhibiting the immunostimulating effect of the trehalose compound (1). Any components may be added, as long as they are pharmacologically and pharmaceutically acceptable. Examples of usable components include excipients, binders, dispersants, thickeners, lubricants, pH adjusters, solubilizing agents and like carriers generally used for the production of preparations. Other examples of usable components include antibiotics, antimicrobial agents, disinfectants, antiseptics, builders, bleaching agents, enzymes, chelating agents, defoaming agents, coloring agents (coloring matter, pigment, etc.), softening agents, moisturizers, surfactants, antioxidants, flavorings, corrigents, odor-masking agents, solvents, etc.

The preparation of the present invention may be taken into the body by oral administration, intravenous drip, injection, etc.; or may be locally administered directly to the affected area.

The appropriate dose of the preparation of the present invention cannot be generalized, because it depends on the dosage form, administration route, etc. The appropriate dose per day per kg of body weight of an adult can be suitably selected depending on the age, condition of the patient, etc.; for example, the appropriate dose is generally 1 ng to 100 mg, and preferably about 10 ng to 50 mg calculated as the weight of the trehalose compound of the present invention. It is preferable that these preparations be divided, and administered several times per day.

### [Advantageous Effects of Invention]

The trehalose compound of the present invention exhibits immunostimulating activity remarkably superior to that of TDCM. The trehalose compound of the present invention has low toxicity, and can therefore be suitably used for treating cancer.

In the present invention, immunostimulating activity includes macrophage activation, neutrophil activation, and phagocytosis activation by phagocytes attributable to the macrophage activation and neutrophil activation, etc.

Macrophage activation indicates the condition wherein the adhesion to the tissue and the motility of the macrophages are improved, so that bacteria entering from outside or denatured self-components are phagocytized. Macrophage activity can be measured using the release of cytokine or active oxygen as an index. In Experimental Example 1 below, the macrophage activation ability was measured using the nitrite ion concentration as an index. The measurement of the nitrite ion concentration was conducted by having the test compound act on the intraperitoneal macrophage cells of a mouse, and measuring the emission of nitrogen monoxide (NO) produced by the activation of macrophages as the nitrite ion concentration within the culture medium.

Neutrophil activation indicates the condition wherein the phagocytosis of neutrophils is accelerated so as to capture bacteria etc. The neutrophil activation can be evaluated using, as an index, the liberation of cytokine or active oxygen, or the liberation of biologically active substance due to degranulation of fine granules or azurophil granules. In Experimental Example 3, the degree of neutrophil activity was analyzed based on the liberation of hydrogen peroxide due to active oxygen (Experimental Example 3) and the phagocytic capacity of *Escherichia coli* (Experimental Example 4). The measurement of hydrogen peroxide was conducted by having the test compound act on rabbit neutrophil, and measuring the emission of hydrogen peroxide produced by the activation of the neutrophil (Experimental Example 3). The phagocytic capacity of *E. coli* was measured by having the test compound act on rabbit neutrophil, phagocytosing the opsonized *E. coli,* and then counting the phagocytosed *E. coli* number (Experimental Example 4).

In order to analyze the effect against the microbism of the test compound, the number of surviving mice was counted after administering the test compound in the abdominal cavity of mice, followed by infection with *Clostridium perfringens* (Experimental Example 2).

From these test results, it became clear that the compound of the present invention has excellent ability of activation of macrophage (phagocytosis) and neutrophil (superoxide generation). Therefore, the compound of the present invention can be suitably used for stimulating macrophage activity, neutrophil activity and phagocytosis activity accompanied by the activation of macrophage activity and neutrophil activity.

Because the production method of the present invention uses a simple reaction without an asymmetric synthesis, the method is advantageous in respect to mass production on an industrial scale.

### BEST MODE FOR CARRYING OUT THE INVENTION

The present invention is explained in detail below with reference to Production Examples and Experimental Examples of the trehalose compound of the present invention (1) and Reference Examples. However, the scope of the present invention is not limited to these examples.

Reference Example 1

### Production of 2-tridecylpentadecanoic acid

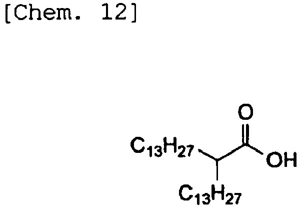

Anhydrous THF (11 ml) was placed in a dried two-neck flask, and sodium hydride (60 w/w%, 397 mg, 9.93 mmol) was added thereto. The mixture was cooled to 0°C, and diethyl malonate (530 mg, 3.31 mmol) was added dropwise. The mixture was then stirred at 0°C for 10 minutes, and 1-iodotridecane (2.56 g, 8.28 mmol) was added thereto, followed by stirring at room temperature for 6 hours. A saturated ammonium chloride solution was added to the resulting mixture, and extraction with diethyl ether was conducted three times. The thus-obtained organic layer was dried over anhydrous magnesium sulfate and concentrated after filtration. The resulting residue was dissolved in a mixed solvent of a 10 N sodium hydroxide aqueous solution (4 ml) and *n-*butanol (8 ml), and then heated under reflux for 6 hours. Subsequently, the reaction mixture was cooled to room temperature, and 1 N hydrochloric acid was added thereto, followed by extraction using diethyl ether repeated three times. The thus-obtained organic layer was dried over anhydrous sodium sulfate, and concentrated after filtration. The resulting residue was dissolved in acetic acid (3.3 ml), heated under reflux for 18 hours, and concentrated under reduced pressure after cooling to remove acetic acid. The residue was purified using silica gel column chromatography (*n*-hexane : ethyl acetate = 7 : 1), and amorphous white powder 2-tridecyl pentadecanoic acid (731 mg, 52%) was obtained.
White powder
FT IR (neat) 3032, 2922, 2851, 2691, 1712 cm⁻¹
¹H NMR (300 MHz, CDCl₃) δ 0.88 (6H, t, J = 6.9Hz), 1.25 (44H, m), 1.48 (2H, m), 1.61 (2H, m), 2.35 (1H, m)
¹³C NMR (75 MHz, CDCl₃) δ 14.14, 22.72, 27.40, 29.39, 29.50, 29.60, 29.64, 29.69, 31.96, 32.19, 45.54, 182.79
CIMS m/z(%) 425 (100 M⁺+1), 423 (30), 242 (19)
Value calculated as HRMS (CI⁺) m/z C₂₈H₅₇O₂ (M⁺+1): 425.4358, Actual measurement value: 425.4341.

Reference Example 2

### Production of 2-undecyl tridecanoic acid

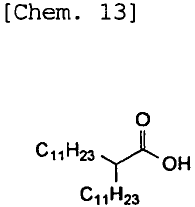

Amorphous white powder 2-undecyl tridecanoic acid was produced in the same manner as in Reference Example 1, using appropriate starting materials.
White powder
FT IR (neat) 3028, 2941, 2858, 1712 cm⁻¹
¹H NMR (300 MHz, CDCl₃) δ 0.88 (6H, t, J = 6.9Hz), 1.25 (36H, m), 1.48 (2H, m), 1.61 (2H, m), 2.35 (1H, m)
¹³C NMR (75 MHz, CDCl₃) δ 14.17, 22.74, 27.41, 29.40, 29.51, 29.61, 29.65, 29.69, 29.72, 31.97, 32.20, 45.55, 182.81
CIMS m/z(%) 369 (100 M⁺+1), 341 (30), 214 (12)
Value calculated as HRMS (CI⁺) m/z C₂₄H₄₉O₂ (M⁺+1) : 369.3732, Actual measurement value: 369.3731.

Reference Example 3

### Production of 2-dodecyl tetradecanoic acid

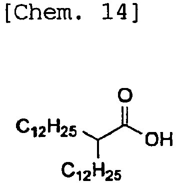

Amorphous white powder 2-dodecyl tetradecanoic acid was produced in the same manner as in Reference Example 1, using appropriate starting materials.
White powder
FT IR (neat) 3028, 2943, 2860, 2691, 1714 cm⁻¹
¹H NMR (300 MHz, CDCl₃) δ 0.88 (6H, t, J = 7.1Hz), 1.25 (40H, m), 1.48 (2H, m), 1.61 (2H, m), 2.34 (1H, m)
¹³C NMR (75 MHz, CDCl₃) δ 14.16, 22.76, 27.43, 29.43, 29.54, 29.64, 29.68, 29.71, 29.72, 31.99, 32.22, 45.68, 183.46
CIMS m/z(%) 397 (100 M⁺+1), 369 (20), 341 (10), 228 (18)
Value calculated as HRMS (CI⁺) m/z C₂₆H₅₃O₂ (M⁺+1) : 397.4045, Actual measurement value: 397.4043.

Reference Example 4

### Production of 2-tetradecyl hexadecanoic acid

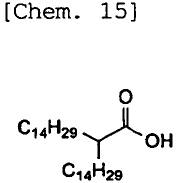

Amorphous white powder 2-tetradecyl hexadecanoic acid was produced in the same manner as in Reference Example 1, using appropriate starting materials.
White powder
FT IR (neat) 3028, 2928, 2854, 2684, 1706 cm⁻¹
¹H NMR (300 MHz, CDCl₃) δ 0.88 (6H, t, J = 7.2Hz), 1.25 (48H, m), 1.48 (2H, m), 1.60 (2H, m), 2.34 (1H, m)
¹³C NMR (75 MHz, CDCl₃) δ 14.13, 22.72, 27.40, 29.40, 29.50, 29.60, 29.64, 29.73, 31.96, 32.19, 45.57, 182.87
CIMS m/z(%) 453 (100 M⁺+1), 452 (45 M⁺), 256 (27), 58 (28)
Value calculated as HRMS (CI⁺) m/z C₃₀H₆₁O₂ (M⁺+1): 453.4671, Actual measurement value: 453.4677.

Reference Example 5

### Production of 2-pentadecyl heptadecanoic acid

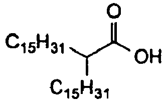

Amorphous white powder 2-pentadecyl heptadecanoic acid was produced in the same manner as in Reference Example 1, using appropriate starting materials.
White powder
FT IR (neat) 3028, 2911, 2848, 2650, 1703 cm⁻¹
¹H NMR (300 MHz, CDCl₃) δ 0.88 (6H, t, J = 6.9Hz), 1.25 (52H, m), 1.48 (2H, m), 1.61 (2H, m), 2.35 (1H, m)
¹³C NMR (75 MHz, CDCl₃) δ 14.05, 22.65, 27.33, 29.33, 29.43, 29.53, 29.57, 29.66, 31.90, 32.14, 45.43, 182.41
CIMS m/z(%) 481 (100 M⁺+1), 480 (40 M⁺)
Value calculated as HRMS (CI⁺) m/z C₃₂H₆₅O₂ (M⁺+1): 481.4984, Actual measurement value: 481.4977.

Reference Example 6

### Production of 2-decyl dodecanoic acid

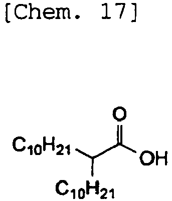

Amorphous white powder 2-decyl dodecanoic acid was produced in the same manner as in Reference Example 1, using appropriate starting materials.
White powder
FT IR (neat) 3041, 2943, 2857, 2689, 1714 cm⁻¹
¹H NMR (300 MHz, CDCl₃) δ 0.88 (6H, t, J = 7.6Hz), 1.21 (32H, m), 1.48 (2H, m), 1.61 (2H, m), 2.34 (1H, m)
¹³C NMR (75 MHz, CDCl₃) δ 14.13, 22.72, 27.40, 29.37, 29.50, 29.64, 31.95, 32.19, 45.61, 183.22
CIMS m/z(%) 341 (100 M⁺+1), 323 (10), 200 (28)
Value calculated as HRMS (CI⁺) m/z C₂₂H₄₅O₂ (M⁺+1): 341.3420, Actual measurement value: 341.3421.

Reference Example 7

### Production of 2-hexadecyl octadecanoic acid

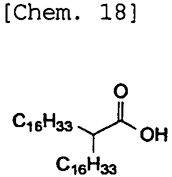

Amorphous white powder 2-hexadecyl octadecanoic acid was produced in the same manner as in Reference Example 1, using appropriate starting materials.
White powder
FT IR (neat) 3028, 2914, 2848, 2691, 1705 cm⁻¹
¹H NMR (300 MHz, CDCl₃) δ 0.88 (6H, t, J = 6.9Hz), 1.25 (56H, m), 1.48 (2H, m), 1.61 (2H, m), 2.35 (1H, m)
¹³C NMR (75 MHz, CDCl₃) δ 14.14, 22.72, 27.40, 29.40, 29.50, 29.60, 29.64, 29.73, 31.96, 32.20, 45.51, 182.51
CIMS m/z(%) 509 (100 M⁺+1), 508 (25 M⁺), 507 (30), 425 (5), 284 (20)
Value calculated as HRMS (CI⁺) m/z C₃₄H₆₉O₂ (M⁺+1): 509.5297, Actual measurement value: 509.5298.

Production Example 1

### (1) 6,6'-bis-N-(2-tridecylpentadecanoylamino)-6,6'-dideoxy 2, 3,4, 2',3',4'-hexabenzyl-α,α'-trehalose

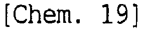

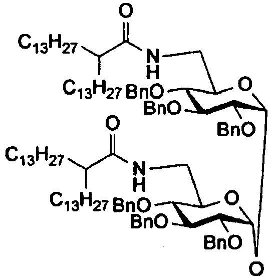

2-Tridecyl pentadecanoic acid (196 mg, 46.2 µmol) and diamino trehalose (6,6'-diamino-2,3,4,2',3',4'-hexa benzyloxy α,α'-trehalose, 163 mg, 18.5 µmol) were dissolved in an anhydrous dichloromethane solution (3 ml). Powder molecular sieves 4A (0.3 g), 4-dimethylaminopyridine (22.6 mg, 18.5 µmol) and 1-ethyl-3-(3-dimethylaminopropyl)carbodiimide hydrochloride (106 mg, 55.5 µmol) were added to the mixture in sequence, and allowed to react at room temperature for 6 hours. The reaction mixture was filtered using Celite-535; distilled water was then added to the filtrate, followed by extraction using dichloromethane repeated three times. The thus-obtained organic layer was dried over anhydrous magnesium sulfate, and concentrated after filtration. The residue was purified using column chromatography (*n*-hexane : ethyl acetate= 5 : 1), and colorless amorphous solid 6,6'-bis-*N-*(2-tridecylpentadecanoylamino)-6,6'-dideoxy-2,3,4,2',3',4'-hexabenzyl-α,α'-trehalose (282 mg, 90 %) was obtained.
Colorless amorphous solid
[α]_{D}²¹ +40.6° (c 0.5 CHCl₃)
FT IR (neat) 3425, 3331, 3088, 3063, 3031, 2932, 2854, 1946, 1874, 1805, 1732, 1680 cm⁻¹¹
¹H NMR (300 MHz, CDCl₃) δ 0.88 (12H, t, J = 6.7Hz), 1.23 (88H, m), 1.34 (4H, m), 1.53 (4H, m), 1.83 (2H, m), 3.00 (2H, dt, J= 14.0Hz, 3.2Hz), 3.30 (2H, t, J = 9.1Hz), 3.47 (2H, dd, J = 9.1Hz, 3.6Hz), 3.91 (2H, ddd, J = 14.0Hz, 9.1Hz, 3.2Hz), 4.04 (2H, t, J = 9.1Hz), 4.10 (2H, m), 4.62 (2H, d, J= 12.0Hz), 4.64 (2H, d, J = 10.3Hz), 4.73 (2H, d, J = 12.0Hz), 4.81 (2H, d, J = 10.3Hz), 4.89 (2H, d, J = 11.0Hz), 4.97 (2H, d, J = 11.0Hz), 5.09 (2H, d, J = 3.6Hz), 5.30 (NH, br d, J = 6.0Hz), 7.22-7.40 (30H, m)
¹³C NMR (75 MHz, CDCl₃) δ 14.11, 22.68, 27.75, 27.88, 29.36, 29.61, 29.71, 31.91, 32.89, 33.04, 38.55, 48.26, 69.67, 73.21, 75.31, 75.57, 78.37, 79.49, 81.53, 93.95, 127.32, 127.56, 127.79, 127.94, 128.37, 128.49, 128.58, 137.89, 138.05, 138.61, 175.94.

(2) Production of 6,6'-bis-*N*-(2-tridecylpentadecanoylamino)-6,6'-dideoxy-α,α'-trehalose

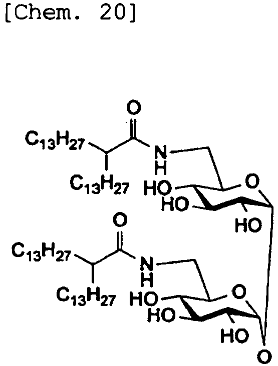

6,6'-Bis-*N*-(2-tridecylpentadecanoylamino)-6,6'-dideoxy-2,3,4,2',3',4'-hexabenzyl-α,α'-trehalose (170 mg, 10 µmol) was dissolved in a mixed solvent (1.7 ml) of chloroform : methanol : acetic acid (1 : 1 : 0.1). Palladium hydroxide (20 w/w%, 7.0 mg, 1.0 µmol) was added thereto and stirred under a 1 atm hydrogen atmosphere for 6 hours. The reaction mixture was concentrated after filtration, and the residue was then purified using column chromatography (dichloromethane : methanol = 7 : 1); colorless amorphous solid 6,6'-bis-*N*-(2-tridecylpentadecanoylamino)-6,6'-dideoxy-α,α'-trehalose (105 mg, 91 %) was obtained.
Colorless amorphous solid
[α]_{D}¹⁹ +38.3° (c 3.1 CHCl₃)
FT IR (neat) 3337, 2931, 2854, 1644 cm⁻¹
¹H NMR (300 MHz, C₅D₅N) δ 0.88 (12H, t, J = 6.9Hz), 1.25 (80H, m), 1.52 (12H, m), 1.93 (4H, m), 2.54 (2H, m), 3.91 (2H, m), 3.97 (2H, t, J = 9.4Hz), 4.23 (2H, dd, J = 9.4Hz, 3.7Hz), 4.36 (2H, m), 4.66 (2H, t, J = 9.4Hz), 4.92 (2H, m), 5.75 (2H, d, J = 3.7Hz), 8.67 (NH, br t, J = 5.1Hz)
¹³C NMR (75 MHz, C₅D₅N) δ 14.24, 22.90, 28.06, 28.16, 29.59, 29.82, 29.94, 30.11, 32.09, 33.55, 33.65, 40.96, 47.59, 74.05, 96.46, 177.55
FABMS m/z(%) 1176 (28 M⁺+Na), 1006 (2), 824 (2), 569 (28), 154 (52), 55 (100)
Value calculated as HRMS (FAB⁺) m/z C₆₈H₁₃₂O₁₁N₂Na (M⁺+Na): 1175.9729, Actual measurement value: 1175.9758.

Production Example 2

### (1) Production of 6,6'-bis-N-(2-undecyltridecanoylamino)-6,6'-dideoxy-2,3,4,2',3',4'-hexabenzyl-α,α'-trehalose

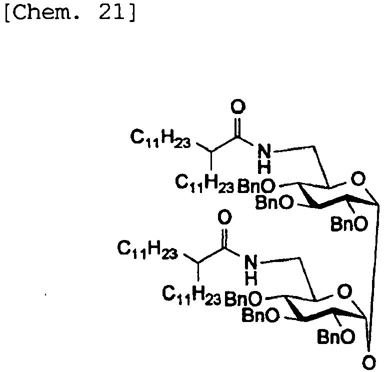

Colorless amorphous solid 6,6'-bis-*N*-(2-undecyltridecanoylamino)-6,6'-dideoxy-2,3,4,2',3',4'-hexabenzyl-α,α'-trehalose was obtained in the same manner as in (1) of Production Example 1.
Colorless amorphous solid
[α]_{D}²¹ +41.8° (c 0.5 CHCl₃)
FT IR (neat) 3426, 3330, 3088, 3063, 3031, 2934, 2854, 1946, 1868, 1806, 1732, 1680 cm⁻¹
¹H NMR (300 MHz, CDCl₃) δ 0.87 (6H, t, J = 6.9Hz), 0.88 (6H, t, J = 6.9Hz), 1.23 (72H, m), 1.34 (4H, m), 1.53 (4H, m), 1.82 (2H, m), 3.00 (2H, dt, J= 14.2Hz, 3.2Hz), 3.30 (2H, t, J = 9.2Hz), 3.47 (2H, dd, J = 9.2Hz, 3.5Hz), 3.92 (2H, ddd, J = 14.2Hz, 9.2Hz, 3.2Hz), 4.04 (2H, t, J = 9.2Hz), 4.10 (2H, m), 4.62 (2H, d, J= 12.0Hz), 4.64 (2H, d, J = 10.2Hz), 4.73 (2H, d, J = 12.0Hz), 4.81 (2H, d, J = 10.2Hz), 4.89 (2H, d, J = 11.0Hz), 4.97 (2H, d, J = 11.0Hz), 5.08 (2H, d, J = 3.5Hz), 5.29 (NH, br d, J = 6.1Hz), 7.22-7.40 (30H, m)
¹³C NMR (75 MHz, CDCl₃) δ 14.12, 22.69, 27.76, 27.88, 29.37, 29.61, 29.68, 31.93, 32.89, 33.04, 38.57, 48.26, 69.67, 73.21, 75.31, 75.58, 78.37, 79.49, 81.54, 93.95, 127.34, 127.57, 127.80, 127.95, 128.39, 128.50, 128.59, 137.89, 138.05, 138.62, 175.97.

(2) Production of 6,6'-bis-*N*-(2-undecyltridecanoylamino)-6,6'-dideoxy-α,α'-trehalose

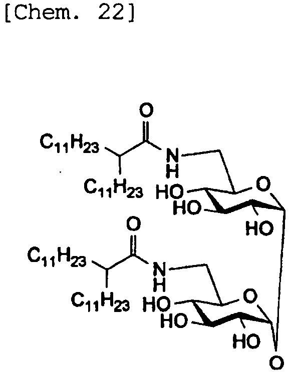

Colorless amorphous solid 6,6'-bis-*N*-(2-undecyltridecanoylamino)-6,6'-dideoxy-α,α'-trehalose was obtained in the same manner as in (2) of Production Example 1.
Colorless amorphous solid
[α]_{D}²⁰ +52.8° (c 0.9 CHCl₃)
FT IR (neat) 3329, 2926, 2853, 1644 cm⁻¹
¹H NMR (300 MHz, C₅D₅N) 5 0.87 (12H, t, J = 7.2Hz), 1.24 (64H, m), 1.52 (12H, m), 1.93 (4H, m), 2.54 (2H, m), 3.91 (2H, m), 3.97 (2H, t, J = 9.5Hz), 4.23 (2H, dd, J = 9.5Hz, 3.8Hz), 4.36 (2H, m), 4.66 (2H, t, J = 9.5Hz), 4.92 (2H, m), 5.75 (2H, d, J = 3.8Hz), 8.67 (NH, br t, J = 5.1Hz)
¹³C NMR (75 MHz, C₅D₅N) 5 14.21, 22.87, 28.04, 28.14, 29.55, 29.78, 29.88, 29.92, 30.08, 32.06, 33.53, 33.64, 40.94, 47.58, 74.04, 96.49, 177.55; FABMS m/z (%) 1064 (55 M⁺+Na), 922 (5), 908 (3), 513 (20), 136 (75), 77 (100)
Value calculated as HRMS (FAB⁺) m/z C₆₀H₁₁₆O₁₁N₂Na (M⁺+Na) : 1063.8477, Actual measurement value: 1063.8505.

Production Example 3

### (1) Production of 6,6'-bis-N-(2-dodecyltetradecanoylamino)-6,6'-dideoxy-2,3,4,2',3',4'-hexabenzyl-α,α'-trehalose

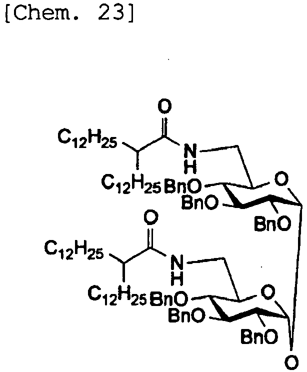

Colorless amorphous solid 6,6'-bis-*N*-(2-dodecyltetradecanoylamino)-6,6'-dideoxy-2,3,4,2',3',4'-hexabenzyl-α,α'-trehalose was obtained in the same manner as in (1) of Production Example 1.
Colorless amorphous solid
[α]_{D}²¹ +41.9° (c 0.5 CHCl₃)
FT IR (neat) 3426, 3334, 3088, 3063, 3031, 2933, 2855, 1946, 1874, 1805, 1731, 1680 cm⁻¹
¹H NMR (300 MHz, CDCl₃) δ 0.87 (6H, t, J = 6.9Hz), 0.88 (6H, t, J = 6.9Hz), 1.23 (80H, m), 1.34 (4H, m), 1.53 (4H, m), 1.82 (2H, m), 3.00 (2H, dt, J= 14.7Hz, 3.2Hz), 3.31 (2H, t, J = 9.4Hz), 3.47 (2H, dd, J = 9.4Hz, 3.6Hz), 3.92 (2H, ddd, J = 14.7Hz, 9.4Hz, 3.2Hz), 4.04 (2H, t, J = 9.4Hz), 4.10 (2H, m), 4.62 (2H, d, J= 12.0Hz), 4.64 (2H, d, J = 10.2Hz), 4.73 (2H, d, J = 12.0Hz), 4.81 (2H, d, J = 10.2Hz), 4.89 (2H, d, J = 11.0Hz), 4.98 (2H, d, J = 11.0Hz), 5.09 (2H, d, J = 3.6Hz), 5.30 (NH, br d, J = 6.0Hz), 7.22-7.40 (30H, m)
¹³C NMR (75 MHz, CDCl₃) δ 14.11, 22.69, 27.75, 27.87, 29.36, 29.60, 29.67, 31.92, 32.89, 30.04, 38.56, 48.26, 69.67, 73.21, 75.31, 75.58, 78.37, 79.49, 81.54, 93.95, 127.33, 127.57, 127.79, 127.95, 128.39, 128.50, 128.59, 137.89, 138.05, 138.62, 175.97.

(2) Production of 6,6'-bis-*N*-(2-dodecyltetradecanoylamino)-6,6'-dideoxy-α,α'-trehalose

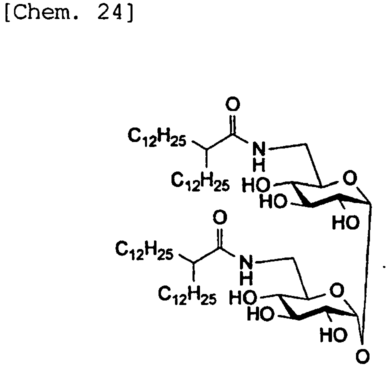

Colorless amorphous solid 6,6'-bis-*N*-(2-dodecyltetradecanoylamino)-6,6'-dideoxy-α,α'-trehalose was obtained in the same manner as in (2) of Production Example 1 (2).
Colorless amorphous solid
[α]_{D}¹⁹ +19.4° (c 1.4 CHCl₃)
FT IR (neat) 3344, 2930, 2854, 1645 cm⁻¹
¹H NMR (300 MHz, C₅D₅N) δ 0.87 (12H, t, J = 7.4Hz), 1.24 (72H, m), 1.52 (12H, m), 1.93 (4H, m), 2.54 (2H, m), 3.91 (2H, m), 3.97 (2H, t, J = 9.3Hz), 4.23 (2H, dd, J = 9.3Hz, 3.9Hz), 4.36 (2H, m), 4.66 (2H, t, J = 9.3Hz), 4.92 (2H, m), 5.75 (2H, d, J = 3.9Hz), 8.67 (NH, br t, J = 5.1Hz)
¹³C NMR (75 MHz, C₅D₅N) δ 14.21, 22.87, 28.03, 28.14, 29.54, 29.78, 29.86, 30.07, 32.05, 33.54, 33.64, 40.94, 47.57, 74.04, 96.49, 177.55
FABMS m/z (%) 1120 (48 M⁺+Na), 964 (5), 950 (3), 541 (20), 523 (14), 136 (80), 77 (100)
Value calculated as HRMS (FAB⁺) m/z C₆₄H₁₂₄O₁₁N₂Na (M⁺+Na): 1119.9103, Actual measurement value: 1119.9077.

Production Example 4

### (1) Production of 6,6'-bis-N-(2-tetradecylhexadecanoylamino)-6,6'-dideoxy-2,3,4,2',3',4'-hexabenzyl-α,α'-trehalose

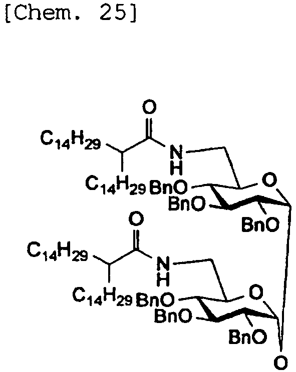

Colorless amorphous solid 6,6'-bis-*N*-(2-tetradecylhexadecanoylamino)-6,6'-dideoxy-2,3,4,2',3',4'-hexabenzyl-α,α'-trehalose was obtained in the same manner as in (1) of Production Example 1.
Colorless amorphous solid
[α]_{D}²¹ +42.6° (c 1. 0 CHCl₃)
FT IR (neat) 3425, 3330, 3088, 3063, 3031, 2932, 2854, 1945, 1870, 1805, 1740, 1680 cm⁻¹
¹H NMR (300 MHz, CDCl₃) δ 0.88 (12H, t, J = 6.9Hz), 1.23 (96H, m), 1.34 (4H, m), 1.53 (4H, m), 1.83 (2H, m), 2.99 (2H, dt, J= 14.3Hz, 3.0Hz), 3.31 (2H, t, J = 9.3Hz), 3.47 (2H, dd, J = 9.3Hz, 3.6Hz), 3.94 (2H, ddd, J = 14.3Hz, 9.3Hz, 3.0Hz), 4.04 (2H, t, J = 9.3Hz), 4.10 (2H, m), 4.62 (2H, d, J= 12.1Hz), 4.64 (2H, d, J = 10.2Hz), 4.73 (2H, d, J = 12.1Hz), 4.81 (2H, d, J = 10.2Hz), 4.90 (2H, d, J = 11.0Hz), 4.98 (2H, d, J = 11.0Hz), 5.08 (2H, d, J = 3.6Hz), 5.30 (NH, br d, J = 6.0Hz), 7.22-7.40 (30H, m)
¹³C NMR (75 MHz, CDCl₃) δ 14.15, 22.70, 27.76, 27.89, 29.37, 29.62, 29.72, 31.92, 32.90, 33.06, 38.50, 48.26, 69.63, 73.17, 75.32, 75.57, 78.29, 79.43, 81.52, 93.97, 127.28, 127.54, 127.76, 127.94, 128.36, 128.46, 128.59, 137.84, 137.96, 138.55, 175.90.

(2) Production of 6,6'-bis-*N*-(2-tetradecylhexadecanoylamino)-6,6'-dideoxy-α,α'-trehalose

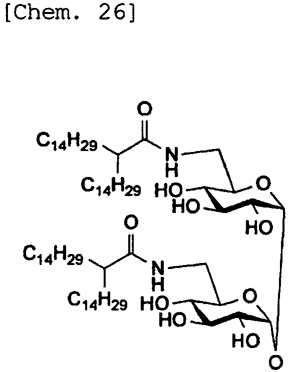

Colorless amorphous solid 6,6'-bis-*N*-(2-tetradecylhexadecanoylamino)-6,6'-dideoxy-α,α'-trehalose was obtained in the same manner as in (2) of Production Example 1.
Colorless amorphous solid
[α]_{D}¹⁹ +36.3° (c 2.1 CHCl₃)
FT IR (neat) 3342, 2937, 2857, 1644 cm⁻¹
¹H NMR (300 MHz, C₅D₅N) δ 0.87 (6H, t, J = 5.8Hz), 0.88 (6H, t, J = 7.2Hz), 1.27 (88H, m), 1.52 (12H, m), 1.93 (4H, m), 2.54 (2H, m), 3.93 (2H, m), 3.97 (2H, t, J = 9.3Hz), 4.23 (2H, dd, J= 9.3Hz, 3.7Hz), 4.36 (2H, m), 4.66 (2H, t, J= 9.3Hz), 4.92 (2H, m), 5.75 (2H, d, J= 3.7Hz), 8.67 (NH, br t, J = 5.1Hz)
¹³C NMR (75 MHz, C₅D₅N) δ 14.22, 22.88, 28.05, 28.15, 29.57, 29.81, 29.94, 30.10, 32.08, 33.54, 33.64, 40.94, 47.58, 74.04, 96.48, 177.53
FABMS m/z(%) 1232 (30 M⁺+Na), 1210 (8), 597 (42), 154 (60), 55 (100)
Value calculated as HRMS (FAB⁺) m/z C₇₂H₁₄₀O₁₁N₂Na (M⁺+Na): 1232.0355, Actual measurement value: 1232.0305.

Production Example 5

### (1) Production of 6,6'-bis-N-(2-pentadecylheptadecanoylamino)-6,6'-dideoxy-2,3,4,2',3',4'-hexabenzyl-α,α'-trehalose

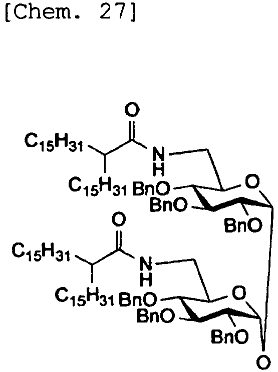

Colorless amorphous solid 6,6'-bis-*N*-(2-pentadecylheptadecanoylamino)-6,6'-dideoxy-2,3,4,2',3',4'-hexabenzyl-α,α-trehalose was obtained in the same manner as in (1) of Production Example 1.
Colorless amorphous solid
[α]_{D}²¹ +38.2° (c 0.5 CHCl₃)
FT IR (neat) 3427, 3333, 3088, 3063, 3031, 2940, 2862, 1945, 1870, 1805, 1740, 1680 cm⁻¹
¹H NMR (300 MHz, CDCl₃) δ 0.88 (12H, t, J = 7.0Hz), 1.23 (104H, m), 1.34 (4H, m), 1.53 (4H, m), 1.83 (2H, m), 2.99 (2H, dt, J = 14.4Hz, 3.3Hz), 3.30 (2H, t, J = 9.4Hz), 3.47 (2H, dd, J= 9.4Hz, 3.6Hz), 3.92 (2H, ddd, J = 14.4Hz, 9.4Hz, 3.3Hz), 4.04 (2H, t, J= 9.4Hz), 4.10 (2H, m), 4.62 (2H, d, J= 12.0Hz), 4.64 (2H, d, J = 10.2Hz), 4.73 (2H, d, J = 12.0Hz), 4.81 (2H, d, J = 10.2Hz), 4.89 (2H, d, J = 11.0Hz), 4.98 (2H, d, J = 11.0Hz), 5.08 (2H, d, J = 3.6Hz), 5.29 (NH, br d, J = 6.0Hz), 7.22-7.40 (30H, m)
¹³C NMR (75 MHz, CDCl₃) δ 14.13, 22.70, 27.76, 27.89, 29.37, 29.72, 31.93, 32.90, 33.05, 38.57, 48.27, 69.68, 73.22, 75.32, 75.58, 78.39, 79.50, 81.55, 93.96, 127.34, 127.58, 127.80, 127.95, 128.39, 128.50, 128.59, 137.90, 138.06, 138.63, 175.96.

(2) Production of 6,6'-bis-*N*-(2-pentadecylheptadecanoylamino)-6,6'-dideoxy-α,α'-trehalose

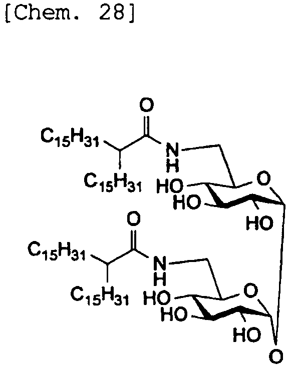

Colorless amorphous solid 6,6'-bis-*N*-(2-pentadecylheptadecanoylamino)-6,6'-dideoxy-α,α'-trehalose was obtained in the same manner as in (2) of Production Example 1.
Colorless amorphous solid
[α]_{D}¹⁹ +32.9° (c 1.2 CHCl₃)
FT IR (neat) 3342, 2928, 2853, 1645 cm⁻¹
¹H NMR (300 MHz, C₅D₅N) δ 0.87 (6H, t, J = 5.6Hz), 0.88 (6H, t, J = 7.0Hz), 1.28 (96H, m), 1.52 (12H, m), 1.93 (4H, m), 2.55 (2H, m), 3.92 (2H, m), 3.97 (2H, t, J = 9.5Hz), 4.23 (2H, dd, J= 9.5Hz, 3.7Hz), 4.36 (2H, m), 4.66 (2H, t, J= 9.5Hz), 4.92 (2H, m), 5.75 (2H, d, J= 3.7Hz), 8.67 (NH, br t, J = 5.0Hz)
¹³C NMR (75 MHz, C₅D₅N) δ 14.20, 22.87, 28.05, 28.14, 29.55, 29.80, 29.87, 29.94, 30.10, 32.06, 33.53, 33.64, 40.94, 47.58, 74.05, 96.49, 177.52
FABMS m/z(%) 1288 (18 M⁺+Na), 625 (20), 607 (12), 154 (55), 55 (100)
Value calculated as HRMS (FAB⁺) m/z C₇₆H₁₄₈O₁₁N₂Na (M⁺+Na): 1288.0980, Actual measurement value: 1288.1003.

Production Example 6

### (1) Production of 6,6'-bis-N-(2-decyldodecanoylamino)-6,6'-dideoxy-2,3,4,2',3',4'-hexabenzyl-α,α'-trehalose

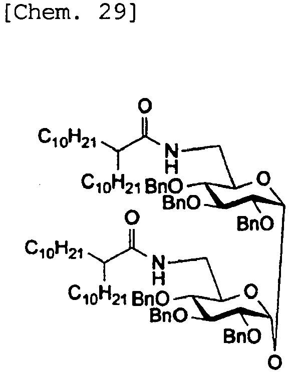

Colorless amorphous solid 6,6'-bis-*N*-(2-decyldodecanoylamino)-6,6'-dideoxy-2,3,4,2',3',4'-hexabenzyl-α,α'-trehalose was obtained in the same manner as in (1) of Production Example 1.
Colorless amorphous solid
[α]_{D}²⁰ +44.6° (c 0.5 CHCl₃)
FT IR (neat) 3426, 3331, 3088, 3063, 3031, 2930, 2853, 1946, 1874, 1806, 1740, 1679 cm⁻¹
¹H NMR (300 MHz, CDCl₃) δ 0.86 (6H, t, J = 7.0Hz), 0.87 (6H, t, J = 7.0Hz), 1.23 (64H, m), 1.34 (4H, m), 1.54 (4H, m), 1.82 (2H, m), 3.00 (2H, dt, J= 14.2Hz, 3.2Hz), 3.30 (2H, t, J = 9.4Hz), 3.47 (2H, dd, J = 9.4Hz, 3.6Hz), 3.93 (2H, ddd, J = 14.2Hz, 9.4Hz, 3.2Hz), 4.04 (2H, t, J = 9.4Hz), 4.10 (2H, m), 4.62 (2H, d, J= 12.0Hz), 4.64 (2H, d, J = 10.2Hz), 4.73 (2H, d, J = 12.0Hz), 4.81 (2H, d, J = 10.2Hz), 4.89 (2H, d, J = 11.0Hz), 4.97 (2H, d, J = 11.0Hz), 5.08 (2H, d, J = 3.6Hz), 5.30 (NH, br d, J = 6.1Hz), 7.22-7.40 (30H, m)
¹³C NMR (75 MHz, CDCl₃) δ 14.11, 22.68, 27.34, 27.87, 29.35, 29.60, 29.68, 29.70, 31.91, 32.89, 33.03, 38.56, 48.25, 69.67, 73.21, 75.31, 75.58, 78.36, 79.48, 81.54, 93.94, 127.33, 127.57, 127.80, 127.95, 128.39, 128.50, 128.58, 137.89, 138.05, 138.62, 175.97.

(2) Production of 6,6'-bis-*N*-(2-decyldodecanoylamino)-6,6'-dideoxy-α,α'-trehalose

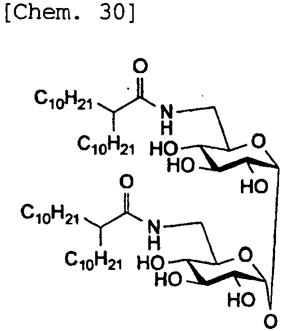

Colorless amorphous solid 6,6'-bis-*N*-(2-decyldodecanoylamino)-6,6'-dideoxy-α,α'-trehalose was obtained in the same manner as in (2) of Production Example 1.
Colorless amorphous solid
[α]_{D}²⁰ +175.7°(c 1.2 CHCl₃)
FT IR (neat) 3346, 2925, 2854, 1645 cm⁻¹¹
¹H NMR (300 MHz, C₅D₅N) δ 0.86 (12H, t, J = 7.6Hz), 1.21 (56H, m), 1.52 (12H, m), 1.92 (4H, m), 2.54 (2H, m), 3.91 (2H, m), 3.97 (2H, t, J = 9.8Hz), 4.23 (2H, dd, J = 9.8Hz, 3.9Hz), 4.36 (2H, m), 4.66 (2H, t, J = 9.8Hz), 4.92 (2H, m), 5.74 (2H, d, J = 3.9Hz), 8.67 (NH, br t, J = 5.1Hz)
¹³C NMR (75 MHz, C₅D₅N) δ 14.22, 22.86, 28.03, 28.14, 29.52, 29.81, 29.89, 29.95, 30.07, 32.05, 33.54, 33.65, 40.94, 47.57, 74.04, 96.49, 177.56
FABMS m/z(%) 1008 (100 M⁺+Na), 880 (10), 740 (10), 524 (12), 484 (32), 136 (100)
Value calculated as HRMS (FAB⁺) m/z C₅₆H₁₀₈O₁₁N₂Na (M⁺+Na): 1007.7851, Actual measurement value: 1007.7846.

Production Example 7

### (1) Production of 6,6'-bis-N-(2-hexadecyloctadecanoylamino)-6,6'-dideoxy-2,3,4,2',3',4'-hexabenzyl-α,α'-trehalose

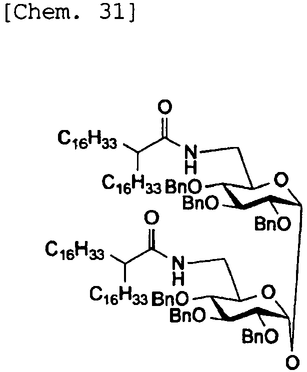

Colorless amorphous solid 6,6'-bis-*N*-(2-hexadecyloctadecanoylamino)-6,6'-dideoxy-2,3,4,2',3',4'-hexabenzyl-α,α'-trehalose was obtained in the same manner as in (1) of Production Example 1.
Colorless amorphous solid
[α]_{D}²¹ +36.0° (c 0.5 CHCl₃)
FT IR (neat) 3426, 3330, 3088, 3063, 3031, 2939, 2857, 1945, 1872, 1805, 1732, 1680 cm⁻¹
¹H NMR (300 MHz, CDCl₃) δ 0.88 (12H, t, J = 7.0Hz), 1.23 (112H, m), 1.34 (4H, m), 1.53 (4H, m), 1.83 (2H, m), 2.99 (2H, dt, J = 14.7Hz, 3.4Hz), 3.30 (2H, t, J = 9.3Hz), 3.47 (2H, dd, J= 9.3Hz, 3.6Hz), 3.92 (2H, ddd, J = 14.7Hz, 9.3Hz, 3.4Hz), 4.04 (2H, t, J= 9.3Hz), 4.09 (2H, m), 4.62 (2H, d, J= 12.0Hz), 4.64 (2H, d, J = 10.0Hz), 4.73 (2H, d, J = 12.0Hz), 4.81 (2H, d, J = 10.0Hz), 4.89 (2H, d, J = 11.0Hz), 4.97 (2H, d, J = 11.0Hz), 5.08 (2H, d, J = 3.6Hz), 5.30 (NH, br d, J = 6.0Hz), 7.22-7.40 (30H, m)
¹³C NMR (75 MHz, CDCl₃) δ 14.10, 22.67, 27.74, 27.87, 29.35, 29.70, 31.91, 32.89, 33.04, 38.55, 48.24, 69.66, 73.20, 75.29, 75.55, 78.36, 79.49, 81.52, 93.92, 127.31, 127.54, 127.77, 127.92, 128.35, 128.47, 128.56, 137.88, 138.04, 138.60, 175.92.

(2) Production of 6,6'-bis-*N*-(2-hexadecyloctadecanoylamino)-6,6'-dideoxy-α,α'-trehalose

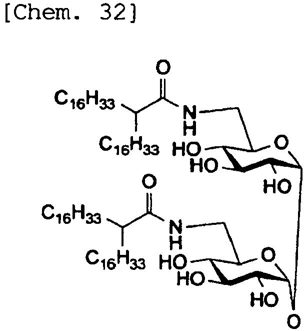

Colorless amorphous solid 6,6'-bis-*N*-(2-hexadecyloctadecanoylamino)-6,6'-dideoxy-α,α'-trehalose was obtained in the same manner as in (2) of Production Example 1.
Colorless amorphous solid
[α]_{D}¹⁹ +37.4° (c 3.0 CHCl₃)
FT IR (neat) 3339, 2929, 2853, 1644 cm⁻¹
¹H NMR (300 MHz, C₅D₅N) δ 0.88 (12H, t, J = 7.0Hz), 1.29 (104H, m), 1.52 (12H, m), 1.93 (4H, m), 2.55 (2H, m), 3.92 (2H, m), 3.97 (2H, t, J = 9.4Hz), 4.23 (2H, dd, J = 9.4Hz, 3.7Hz), 4.36 (2H, m), 4.66 (2H, t, J = 9.4Hz), 4.92 (2H, m), 5.75 (2H, d, J = 3.7Hz), 8.67 (NH, br t, J = 5.1Hz)
¹³C NMR (75 MHz, C₅D₅N) δ 14.24, 22.90, 28.06, 28.16, 29.59, 29.90, 29.98, 30.12, 32.09, 33.54, 33.65, 40.95, 47.59, 74.04, 96.48, 177.52
FABMS m/z (%) 1344 (6 M⁺+Na), 653 (12), 537 (8), 154 (59), 55 (100) Value calculated as HRMS (FAB⁺) m/zC₈₀H₁₅₆O₁₁N₂Na (M⁺+Na): 1344.1607, Actual measurement value: 1344.1633.

Experimental Example 1: Measurement of Microphage Activity

### Experimental Method

### Principle:

The present experiment was conducted with reference to the method described in Sakurai T., Kaise T., Matsubara C. Chem. Res. Toxicol. 1998, 11, 273-283. Specifically, ICR male mice (6 to 7 weeks old) were anesthetized, a phosphate buffer containing 0.05% EDTA was injected intraperitoneally, an intraperitoneal lavage was performed, and a peritoneal macrophage was aseptically recovered. After the cells were washed with an RPMI-1640 medium (containing 10% bovine fetal serum), the macrophage cells were inoculated so that there were 1 × 10⁵ cells/100 µl in each well of the 96-well plate. After being pre-incubated for 2 hours, the test compound (50 µM) was added to the culture medium, and cultured for 48 hours. Afterwards, a Griess test reagent was added to the culture medium, which was allowed to stand for 10 minutes; the absorbency thereof was measured with a microplate reader (filter: 550/630 nm), after which the nitrite ion concentration (NO₂⁻) was determined from a calibration curve. The reproducibility of the above-described test was confirmed by 3 wells, the experiment was repeated at least three times using different mice, and the mean values ± S.E. were determined. Table 1 shows the results.

Moreover, the measurement of the nitrite ion concentration was conducted by having the test compound act on the intraperitoneal macrophage cells of a mouse, and measuring the emission of nitrogen monoxide (NO) produced by the activation of macrophages as the nitrite ion concentration within the culture medium.

**Table 1**

| Test Compound | | R¹=R²= R³=R⁴ | Nitrite Ion Concentration | |
|---|---|---|---|---|
| | | | Actual Value (nM) | Relative Value |
| Ex.1 | 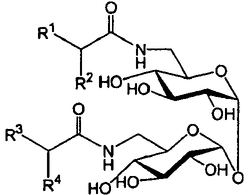 | n-C₁₀H₂₁ | 200±133 | 0.4 |
| Ex.2 | | n-C₁₁H₂₃ | 1973±973 | 3.9 |
| Ex.3 | | n-C₁₂H₂₅ | 1202±647 | 2.4 |
| Ex.4 | | n-C₁₃H₂₇ | 3676±385 | 7.6 |
| Ex.5 | | n-C₁₄H₂₉ | 4419±2450 | 8.8 |
| Ex.6 | | n-C₁₅H₃₁ | 851±608 | 1.7 |
| Ex.7 | | n-C₁₆H₃₃ | 300±229 | 0.6 |
| TDCM | 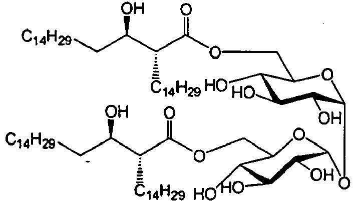 | | 500±271 | 1.0 |

### Experimental Example 2: Influence on Clostridium perfringens-administered mice

The influence on *Clostridium perfringens*-administered mice was measured by the method below. Six-week-old ICR mice (SPF; Charles River Laboratories, Inc.) were employed.

### (1) Preparation of Clostridium perfringens (Type A NTCT8237):

Powdered brain heart infusion (BHI) (7.4 g; Difco) was dissolved in 200 ml distilled water. The obtained solution (40 ml) was collected with a volumetric pipette, added to a 200 ml flask, and high-pressure steam sterilization (121°C, 20 minutes) was conducted. Next, 5 ml of BHI culture medium was placed in two screw cap test tubes, and high-pressure steam sterilization was conducted. Then, a rubber stopper (with cotton plug) with glass tube and the glass tube were sterilized with high-pressure steam.

A proper amount of *Clostridium perfringens* grown in a cooked meat medium was collected with a sterile Pasteur pipette in a clean bench, and grown in a BHI culture medium within a sterile screw cap test tube. Then, it was incubated overnight at 37°C.
In a clean bench, *Clostridium perfringens* grown within the sterile screw cap test tube (the *Clostridium perfringens* growing in the clean bench was cloudy and produced gas) was transferred to a 200 ml Erlenmeyer flask containing 40 ml of BHI culture medium, a glass tube was inserted into the culture medium, and nitrogen substitution was conducted for 10 minutes. Next, the glass tube with rubber stopper (with cotton plug) was mounted on the Erlenmeyer flask (in order for *Clostridium perfringens* to produce a gas, air holes are required), and afterwards incubated at 37°C for 4 to 5 hours. The cultured *Clostridium perfringens* was transferred to a centrifuge tube, centrifugal separation (9,000 rpm, 15 minutes) was conducted, and the supernatant was removed. After the *Clostridium perfringens* was suspended by adding 20 ml of sterile physiological saline to the sediment, centrifugal separation (9,000 rpm, 15 minutes) was conducted, and the supernatant was removed. The obtained sediment was added to the BHI culture medium within the sterile screw cap test tube, and the *Clostridium perfringens* number was measured with a OneCell Counter (OneCell, Inc.).

### (2) Preparation of emulsion solution of trehalose compound and TDCM

A homogenizer (manufactured by Wheaton, Inc.) was prechilled. Trehalose compound or TDCM (1 mg) was weighed, and placed at the bottom portion of the homogenizer using a microspatula. After one drop of mineral oil (Nacalai Tesque, Inc.) was added thereto, the mineral oil and the emulsion solution of trehalose compound or TDCM were mixed with the homogenizer by kneading so as completely dissolve the emulsion solution of trehalose compound or TDCM. Then, 1 ml of physiological saline containing 1.1% Tween 80 and 5.6% mannitol was placed in the homogenizer. Homogenization was conducted for 3 minutes with ice, and the solution was transferred into an Eppendorf tube. Next, low-temperature sterilization was conducted at 62°C for 30 minutes.

### (3) Experimental Method

### In accordance with the procedures below, the influence on Clostridium perfringens-administered mice was measured.

In accordance with the above-mentioned method, 1 mg/ml of the emulsion solution of the compound of Experiment 1, the compound of Experiment 4, the compound of Experiment 5, and the TDCM were each prepared. The emulsion solution (100 mg/mouse) of the compound of Experiment 1, the compound of Experiment 4, the compound of Experiment 5 and the TDCM, as well as 100 mg/mouse of the emulsion solution alone as a control, were each administered to separate groups of four 6-week-old ICR mice. After 3 hours, *Clostridium perfringens* was administered to the mice at 2.4 × 10⁷ cells/mouse. Visual observations were conducted over time. Table 2 shows the results.

**Table 2**

| Test Compound | The number surviving/Administered group | | | | |
|---|---|---|---|---|---|
| | After 2 hr | After 6 hr | After 12 hr | After 24 hr | after 48 hr |
| Ex.1 | 4/4 | 4/4 | 4/4 | 4/4 | 4/4 |
| Ex.4 | 4/4 | 4/4 | 4/4 | 4/4 | 4/4 |
| Ex.5 | 4/4 | 4/4 | 4/4 | 4/4 | 4/4 |
| TDCM | 4/4 | 4/4 | 4/4 | 4/4 | 4/4 |
| Emulsion solution alone (Control) | 4/4 | 3/4 | 0/4 | 0/4 | 0/4 |

### Experimental Example 3: Influence on rabbit neutrophil H₂O₂ production

The materials below were employed when measuring the influence on rabbit neutrophil H₂O₂ production.

### (1) Preparation of rabbit neutrophil suspension solution Preparation of Hanks' balanced salt solution (HBS):

After 0.4 g of potassium chloride, 0.06 g of potassium dihydrogen phosphate, 0.107 g of disodium hydrogen phosphate, and 8 g of sodium chloride were dissolved with distilled water (hereunder may be referred to as D.W.), a 1 N sodium hydroxide aqueous solution was prepared at a pH of 7.4, and brought to a total volume of 1,000 ml with D.W.

Preparation of HBS (HBSG-BSA) containing 5.5 mM of glucose and 0.003% BSA:
Glucose (0.1 g) and 0.003 g of BSA (Sigma, Inc.; A-1498) were dissolved in 100 ml of HBS (*in situ*).

Preparation of citric acid-glucose solution (ACD solution):
Sodium citrate (6.25 g), 3.125 g of citric acid, and 5 g of glucose were dissolved with 250 ml of D.W., and stored at 4°C until used.

Preparation of erythrocyte lysis solution (lysis solution):
EDTA (0.037 g), 1 g of potassium hydrogen carbonate, and 8.3 g of ammonium chloride were dissolved with 1,000 ml of D.W., and stored at 4°C until used.
Preparation of phosphate-buffered saline (PBS):
Sodium chloride (8.0 g), 0.2 g of potassium chloride, 1.15 g of disodium hydrogen phosphate, and 0.2 g of potassium dihydrogen phosphate were dissolved with 1,000 ml of D.W.

Preparation of 1.2% dextran-PBS solution:
Dextran T500 (1.2 g) (Pharmacia, 17-0320-01) was dissolved with 100 ml of D.W., sterilized with an autoclave (121°C, 20 minutes), and stored at 4°C until used.

### Method for preparation of rabbit neutrophil suspension solution:

ACD solution (5 ml) was drawn into a 20 ml syringe (hypodermic needle; Nipro), and the inside of the syringe was uniformly rinsed. After 20 ml of blood was collected from the medial auricular artery of a rabbit, the blood was gently mixed by inversion, and dispensed into three centrifuge tubes (15 ml type; Falcon); after centrifugation for 5 minutes at 1,500 rpm at 4°C, the supernatant was collected from the centrifuge tubes (50 ml type; Falcon). After 1.2% dextran-PBS solution was added in an amount equal to that of the collected supernatant, and gently mixed by inversion, the solution was allowed to stand for 30 minutes at room temperature. The interface was confirmed, and the supernatant was placed in a fresh centrifuge tube (50 ml type; Falcon). The same amount of 1.2% dextran-PBS solution was added to the residual solution and gently mixed by inversion, and allowed to stand for 30 minutes at room temperature. The interface was confirmed, and the supernatant was placed in a centrifuge tube (50 ml type; Falcon). The collected supernatant was removed after being centrifuged for 10 minutes at 2,000 rpm at 4°C. After 15 ml of the lysis solution was added to the sediment and suspended gently, an additional 5 ml of lysis solution was added and gently mixed by inversion, and then allowed to sit in ice for 5 minutes. After the total volume was raised to 50 ml with HBSG-BSA and centrifugation was performed for 10 minutes at 2,000 rpm at 4°C, the supernatant was removed. The sediment was suspended with 2 ml of HBSG-BSA; this cellular suspension was gently overlaid on a 2 ml top layer (centrifuge tube, 15 ml type) of Lymphoprep (Nycomed, 808068), and after being centrifuged for 20 minutes at 1,200 rpm (centrifuge conditions: accel 0.5, break off), the supernatant was removed with an aspirator. In order to remove the remaining Lymphoprep, the sediment (neutrophil) was suspended with HBSG-BSA, and the process was then repeated; the solution was centrifuged for 5 minutes at 1,500 rpm, and the supernatant was removed. The neutrophil was suspended with HBSG-BSA, and a cell count was performed with a "Celltac" cell-counting device (Nihon Kohden Corporation).

### (2) Preparation of trehalose compound and TDCM emulsion solution

A potter homogenizer (Wheaton, Inc.) was chilled for 3 minutes with ice. One milligram of the trehalose compound or TDCM was weighed, and placed at the bottom of the chilled homogenizer. Next, 500 µl of 2% BSA was added (2 g of BSA (Sigma) was dissolved with 100 ml of physiological saline), and the solution was homogenized until suspended (approximately 5 minutes). The obtained suspension solution was transferred to an Eppendorf tube, and treated with ultrasound for 3 minutes (150 W).

### (3) Preparation of 10 mM H₂DCFDA (2', 7'-dichlorodihydrofluorescein diacetate (Molecular Probes, D399)):

H₂DCFDA (4.86 mg) was dissolved with 1 ml of DMSO.

### (4) Experimental Method

The influence on rabbit neutrophil H₂O₂ production was measured by the below-mentioned procedures.

The neutrophils (1.0 x 10⁵ cells/100 µl) were inoculated in a 96-well plate (Falcon). One microliter of 10 µM H₂DCFDA was added thereto, and the solution was incubated for 1 hour at 37°C. After suspension by adding 300 µl of HBS in order to remove the excess H₂DCFDA, the solution was centrifuged for 5 minutes at 8,000 rpm. The supernatant was removed and suspended with HBS, and then HBS was added to bring the final concentration thereof to 50 µM. This solution was incubated for 2 hours at 37°C, after which the H₂O₂ production was measured with a fluorescent measuring device (Ex: 485 nm, Em: 535 nm). Table 3 shows the results.

Moreover, the degree of neutrophil activity may be determined by measuring the amount of hydrogen peroxide (H₂O₂) derived from superoxides (O²⁻) produced by neutrophils. The above amount of hydrogen peroxide indicates the hydrogen peroxide-producing capacity of the neutrophils, and thus is capable of indicating the degree of neutrophil activity as an activity indicator.

**Table 3**

| Test Compound | H₂O₂ production (nmol/10⁵Cells) | |
|---|---|---|
| | 50µM | 100µM |
| Ex.1 | 0.33±0.05 | 0.38±0.05 |
| Ex.2 | 0.25±0.02 | 0.26±0.02 |
| Ex.3 | 0.18±0.03 | 0.20±0.03 |
| Ex.4 | 0.15±0.02 | 0.18±0.02 |
| Ex.5 | 0.26±0.04 | 0.29±0.04 |
| Ex. 6 | 0.11±0.03 | 0.16±0.03 |
| Ex.7 | 0.10±0.03 | 0.15±0.02 |
| 2%BSA(Control) | 0.08±0.01 | 0.11±0.02 |
| TDCM | 0.18±0.02 | 0.21±0.02 |

### Experimental Example 4: Influence of the trehalose compound on the phagocytic action of neutrophils

The influence of the trehalose compound on the phagocytic action of neutrophils was measured by the below-mentioned method.

### (1) Preparation rabbit neutrophil suspension solution:

Preparation of Hanks' balanced salt solution (HBS):
After 0.4 g of potassium chloride, 0.06 g of potassium dihydrogen phosphate, 0.107 g of disodium hydrogen phosphate, and 8 g of sodium chloride were dissolved with distilled water (D.W.), a 1 N sodium hydroxide aqueous solution was prepared at a pH of 7.4, and brought to a total volume of 1,000 ml with D.W.

Preparation of HBS (HBSG-BSA) containing 5.5 mM of glucose and 0.003% BSA:
Glucose (0.1 g) and 0.003 g of BSA (Sigma, Inc.; A-1498) were dissolved in 100 ml of HBS (*in situ*).

Preparation of citric acid-glucose solution (ACD solution):
Sodium citrate (6.25 g), 3.125 g of citric acid, and 5 g of glucose were dissolved with 250 ml of D.W., and stored at 4°C until used.

Preparation of erythrocyte lysis solution (lysis solution):
EDTA (0.037 g), 1 g of potassium hydrogen carbonate, and 8.3 g of ammonium chloride were dissolved with 1,000 ml of distilled water, and stored at 4°C until used.

Preparation of phosphate-buffered saline (PBS):
Sodium chloride (8.0 g), 0.2 g of potassium chloride, 1.15 g of disodium hydrogen phosphate, and 0.2 g of potassium dihydrogen phosphate were dissolved with 1,000 ml of distilled water.

Preparation of 1.2% dextran-PBS solution:
Dextran T500 (1.2 g) (Pharmacia, 17-0320-01) was dissolved with 100 ml of D.W., sterilized with an autoclave (121°C, 20 minutes), and stored at 4°C until used.

### Method for preparation of rabbit neutrophil suspension solution:

ACD solution (5 ml) was drawn into a 20 ml syringe (hypodermic needle; Nipro), and the inside of the syringe was uniformly rinsed. After 20 ml of blood was collected from the medial auricular artery of a rabbit, the solution was gently mixed by inversion, dispensed into three centrifuge tubes (15 ml type; Falcon), and after centrifugation for 5 minutes at 1,500 rpm at 4°C, the supernatant was collected from the centrifuge tubes (50 ml type; Falcon). After 1.2% dextran-PBS solution was added in an equal amount to that of the collected supernatant and gently mixed by inversion, the solution was allowed to stand for 30 minutes at room temperature. The interface was confirmed, and the supernatant was placed in a fresh centrifuge tube (50 ml type; Falcon). The same amount of 1.2% dextran-PBS solution was added to the residual solution, gently mixed by inversion, then allowed to stand for 30 minutes at room temperature. The interface was confirmed, and the supernatant was placed in a centrifuge tube (50 ml type; Falcon). After the collected supernatant was centrifuged for 10 minutes at 2,000 rpm at 4°C, the supernatant was removed. After 15 ml of lysis solution was added to the sediment and suspended gently, an additional 5 ml of lysis solution was added and gently mixed by inversion, and then allowed to sit in ice for 5 minutes. After the total volume was raised to 50 ml with HBSG-BSA, and centrifugation performed for 10 minutes at 2,000 rpm at 4°C, the supernatant was removed. The sediment was suspended with 2 ml of HBSG-BSA, and this cellular suspension solution was gently overlaid on a 2 ml top layer (centrifuge tube, 15 ml type) of Lymphoprep (Nycomed, 808068); after being centrifuged for 20 minutes at 1,200 rpm (centrifuge conditions: accel 0.5, break off), the supernatant was removed with an aspirator. In order to remove the remaining Lymphoprep, the sediment (neutrophil) was suspended with HBSG-BSA, and the process was then repeated; the solution was centrifuged for 5 minutes at 1,500 rpm, and the supernatant was removed. The neutrophil was suspended with HBSG-BSA, and a cell count was performed with a "Celltac" cell-counting device (Nihon Kohden Corporation).

### (2) Method for preparation of ampicillin resistant Escherichia coli and opsonized Escherichia coli

### Materials:

Ampicillin resistant plasmid (pT7Blue, Novagen);
Competent cells (JM109, TAKARA);
L-broth (10 g of tryptophan, 15 g of NaCl, 5 g of yeast extract, and 1 ml of MgSO₄ per 1 L of D.W.); and
BioParticles Opsonizing reagent (Molecular Probes Lot: 25530W).

### Preparation of BioParticles Opsonizing reagent: 10 mg of opsonizing reagent was dissolved with 500 µl of ultra-pure water.

### Preparation of ampicillin resistant Escherichia coli

Electroporation cells (JM109) were lysed with ice, 2 µl of an ampicillin-resistant plasmid (100 ng/µl) was suspended by addition to the JM109, and the suspension solution thereof was transferred to an electroporation cuvette and a pulse applied thereto (2.5 kV, 200 Q, 25 mF), in order to introduce the ampicillin-resistant gene into *Escherichia coli.* The *Escherichia coli* to which to the pulse was applied was transferred to a 5 ml tube with 1 ml of L-broth placed therein, and cultured for 3 hours at 37°C. The above solution was coated onto an agar medium of L-broth containing 0.005% ampicillin, and cultured overnight at 37°C (when inoculating in a medium, after being centrifuged for 5 minutes at 3,500 rpm, 800 µl of the supernatant was removed, the sediment was suspended, and then inoculated in a petri dish). A small amount of the *Escherichia coli* that had grown the next day was scraped up, lysed in 2 ml of an L-broth medium, and placed in an agitated culture for approximately 4 hours.

### Method for preparation of opsonized Escherichia coli

One hundred microliters of the above-prepared ampicillin-resistant *Escherichia coli* solution and 100 µl of the dissolved opsonizing reagent were suspended within Eppendorf tubes. The obtained suspension solution was incubated for 1 hour at 37°C, suspended with 300 µl of PBS, then centrifuged for 15 minutes at 1,200 G, and afterwards the supernatant was removed. Next, the obtained solution was suspended with 300 µl of PBS, centrifuged for 15 minutes at 1,200 G, and afterwards the supernatant was removed. The above process was performed two times. One microliter of the *Escherichia coli* solution was added to 100 µl of L-broth, thoroughly suspended, and then diluted 100-fold. The 100-fold-diluted *Escherichia coli* solution was added to a 10 µl OneCell Counter, and the *Escherichia coli* number was counted via a microscope.

### (3) Preparation of trehalose compound or TDCM emulsion solution

A potter homogenizer (Wheaton, Inc.) was chilled for 3 minutes with ice. The 1 mg of trehalose compound that was weighed was placed at the bottom of the chilled homogenizer. Five hundred milliliters of 2% BSA (2 g of BSA (Sigma) was dissolved with 100 ml of physiological saline solution) was added, and the solution was homogenized until suspended (approximately 5 minutes). The suspension solution was transferred to an Eppendorf tube, and treated with ultrasound for 3 minutes (150 W).

### (4) Experimental Method

In accordance with the neutrophil collection method, the neutrophil was prepared from rabbit blood, and the number of neutrophil cells was measured. The neutrophils were dispensed at 1.0×10⁵ cells in an Eppendorf tube, suspended with HBS, and then treated for 1 hour with 50 µM of an emulsion solution of trehalose compound or an emulsion solution of TDCM (control 2% BSA). It was suspended by the addition of 300 µl of HBS, centrifuged for 10 minutes at 1,200 G, and afterwards the supernatant was removed. Next, the obtained solution was suspended by the addition of 300 µl of HBS thereto, then centrifuged for 10 minutes at 1,200 G, and afterwards the supernatant was removed. After 1.0×10⁷ cells of the opsonized ampicillin resistant *E. coli* was thoroughly suspended in the treated neutrophils, it was incubated for 1 hour at 37°C. After the excess E. *coli* was removed, 100 µl of 0.5% Triton X-100 (physiological saline) was added, it was suspended thoroughly, and incubated for 30 minutes at 37°C. Next, the total volume of the Triton X-100 treatment solution was inoculated in a normal agar medium containing 0.005% ampicillin, and spread across the entire surface thereof with a bacteria spreader. The incubation thereof was conducted for 24 hours at 37°C, and the number of colonies thereof was counted. Table 4 shows the results.

**Table 4**

| Test Compound | The number of colonies | |
|---|---|---|
| | 50µM | 100µM |
| Ex.1 | 250±11 | 487±20 |
| Ex.2 | 89±21 | 172±13 |
| Ex.3 | 58±9 | 112±15 |
| Ex.4 | 200±15 | 350±30 |
| Ex.5 | 250±16 | 450±31 |
| Ex.6 | 120±11 | 220±12 |
| Ex.7 | 110±7 | 200±8 |
| 2%BSA(Control) | 10±5 | 6±5 |
| TDCM | 50±10 | 87±10 |

## Claims

1. A trehalose compound represented by general formula (1) : 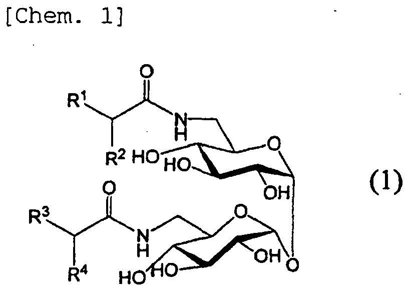 wherein R¹, R², R³ and R⁴ may be the same or different, and independently represent a C₁₀₋₁₆ alkyl group.

2. A process for producing the trehalose compound according to claim 1,
the process comprising the step of removing a protective group for a hydroxyl group from a trehalose compound represented by general formula (5): 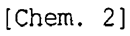 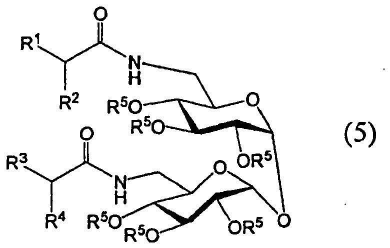 wherein R¹, R², R³ and R⁴ are the same as above, and R⁵ is a protective group for a hydroxyl group.

3. A process for producing the trehalose compound according to claim 1,
the process comprising the steps of:
simultaneously or sequentially reacting a trehalose compound represented by general formula (2): 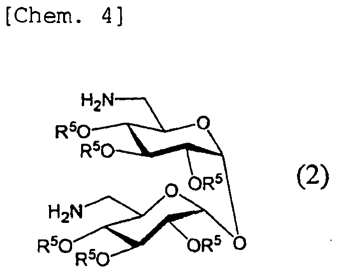 wherein R⁵ represents a protective group for hydroxyl group,
with a carbonyl compound represented by general formula (3) and a carbonyl compound represented by general formula (4): 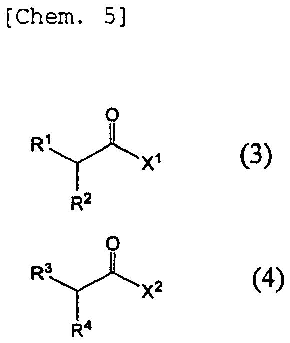
wherein R¹, R², R³ and R⁴ may be the same or different, and independently represent a C₁₀₋₁₆ alkyl group, and X¹ and X² may be the same or different, and independently represent a hydroxyl group or a halogen atom; and
removing a protective group for a hydroxyl group from the resulting trehalose compound represented by general formula (5): 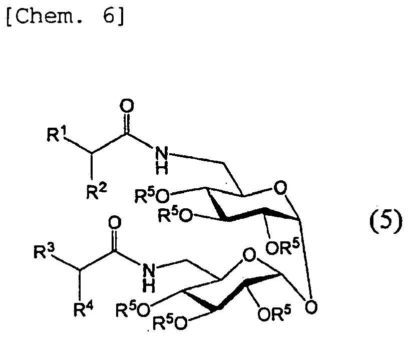 wherein R¹, R², R³, R⁴ and R⁵ are the same as above.

4. An immunostimulating agent comprising the trehalose compound defined by claim 1 as an active ingredient.

5. A macrophage immunostimulating agent comprising the trehalose compound defined by claim 1 as an active ingredient.

6. An immunostimulating agent for stimulating activity of neutrophil comprising the trehalose compound defined by claim 1 as an active ingredient.

7. An immunostimulating agent for stimulating the phagocytosis of phagocytes comprising the trehalose compound defined by claim 1 as an active ingredient.

8. An anti-microbism agent comprising the trehalose compound defined by claim 1 as an active ingredient.

## Patentansprüche

1. Trehaloseverbindung, dargestellt durch die allgemeine Formel (1): 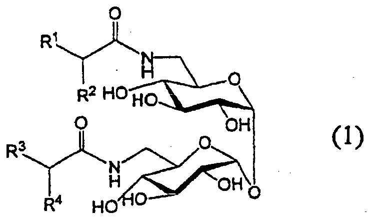 wobei R¹, R², R³ und R⁴ gleich oder verschieden sein können und unabhängig voneinander eine C₁₀₋₁₆-Alkylgruppe darstellen.

2. Verfahren zur Herstellung der Trehaloseverbindung nach Anspruch 1, wobei das Verfahren den Schritt des Entfernens einer Schutzgruppe für eine Hydroxylgruppe von einer Trehaloseverbindung umfasst, dargestellt durch die allgemeine Formel (5): 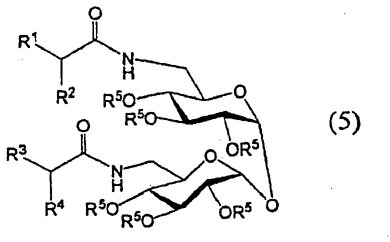 wobei R¹, R², R³ und R⁴ gleich wie vorstehend sind und R⁵ eine Schutzgruppe für eine Hydroxylgruppe ist.

3. Verfahren zur Herstellung der Trehaloseverbindung nach Anspruch 1, wobei das Verfahren die Schritte umfasst:
gleichzeitiges oder nacheinander erfolgendes Umsetzen einer Trehaloseverbindung, dargestellt durch die allgemeine Formel (2): 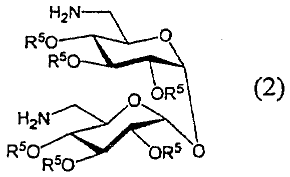 wobei R⁵ eine Schutzgruppe für eine Hydroxylgruppe darstellt,
mit einer Carbonylverbindung, dargestellt durch die allgemeine Formel (3) und einer Carbonylverbindung, dargestellt durch die allgemeine Formel (4): 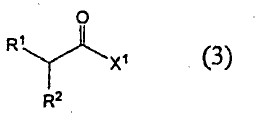 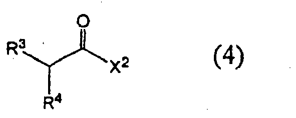
wobei R¹, R², R³ und R⁴ gleich oder verschieden sein können und unabhängig eine C₁₀₋₁₆-Alkylgruppe darstellen, und X¹ und X² gleich oder verschieden sein können, und unabhängig voneinander eine Hydroxylgruppe oder ein Halogenatom darstellen, und
Entfernen einer Schutzgruppe für eine Hydroxylgruppe von der sich ergebenden Trehaloseverbindung, dargestellt durch die allgemeine Formel (5): 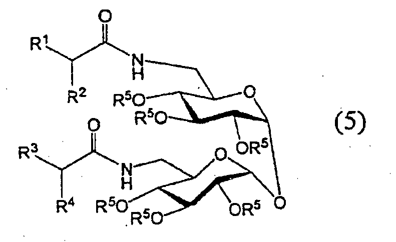 wobei R¹, R², R³, R⁴ und R⁵ gleich wie vorstehend sind.

4. Immunstimulierendes Mittel, umfassend die Trehaloseverbindung wie in Anspruch 1 definiert als aktiven Bestandteil.

5. Makrophagen-immunstimulierendes Mittel, umfassend die Trehaloseverbindung wie in Anspruch 1 definiert als aktiven Bestandteil.

6. Immunstimulierendes Mittel für das Stimulieren der Aktivität von Neutrophilen, umfassend die Trehaloseverbindung wie in Anspruch 1 definiert als aktiven Bestandteil.

7. Immunstimulierendes Mittel für das Stimulieren der Phagocytose von Phagocyten, umfassend die Trehaloseverbindung wie in Anspruch 1 definiert als aktiven Bestandteil.

8. Antimikrobismusmittel, umfassend die Trehaloseverbindung wie in Anspruch 1 definiert als aktiven Bestandteil.

## Revendications

1. Composé tréhalose représenté par la formule générale (1) : 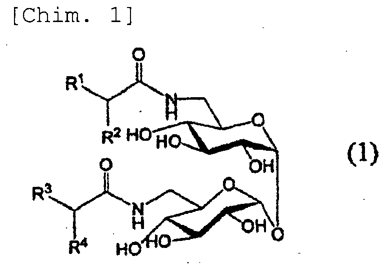 dans laquelle R¹, R², R³ et R⁴ peuvent être identiques ou différents et représentent indépendamment un groupe alkyle en C₁₀-C₁₆.

2. Procédé pour produire le composé tréhalose selon la revendication 1, le procédé comprenant l'étape consistant à éliminer un groupe protecteur d'un groupe hydroxyle d'un composé tréhalose représenté par la formule générale (5) : 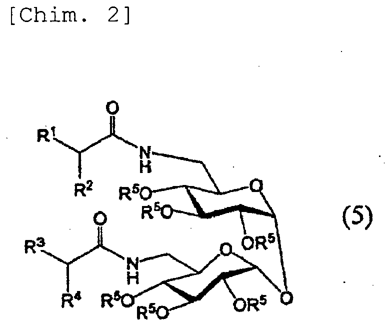 dans laquelle R¹, R², R³ et R⁴ sont les mêmes que ci-dessus et R⁵ est un groupe protecteur d'un groupe hydroxyle.

3. Procédé pour produire le composé tréhalose selon la revendication 1, le procédé comprenant les étapes consistant à :
faire réagir simultanément ou séquentiellement un composé tréhalose représenté par la formule générale (2) : 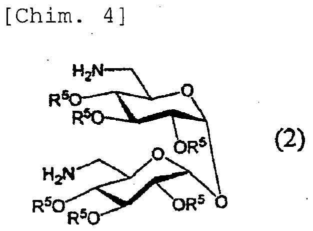 dans laquelle R⁵ représente un groupe protecteur d'un groupe hydroxyle, avec un composé carbonyle représenté par la formule générale (3) et un composé carbonyle représenté par la formule générale (4) : 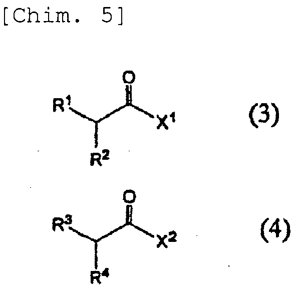
dans laquelle R¹, R², R³ et R⁴ peuvent être identiques ou différents et représentent indépendamment un groupe alkyle en C₁₀-C₁₆, et X¹ et X² peuvent être identiques ou différents et représentent indépendamment un groupe hydroxyle ou un atome d'halogène ; et à
éliminer un groupe protecteur d'un groupe hydroxyle du composé tréhalose obtenu représenté par la formule générale (5) : 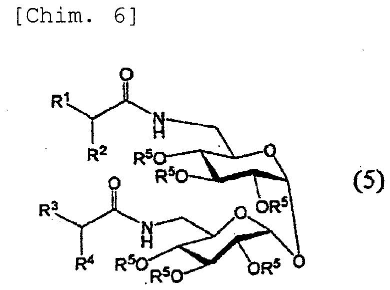 dans laquelle R¹, R², R³, R⁴ et R⁵ sont les mêmes que ci-dessus.

4. Agent immunostimulant comprenant le composé tréhalose défini par la revendication 1 en tant que principe actif.

5. Agent immunostimulant pour activer les macrophages comprenant le composé tréhalose défini par la revendication 1 en tant que principe actif.

6. Agent immunostimulant pour stimuler l'activité des neutrophiles comprenant le composé tréhalose défini par la revendication 1 en tant que principe actif.

7. Agent immunostimulant pour stimuler la phagocytose des phagocytes comprenant le composé tréhalose défini par la revendication 1 en tant que principe actif.

8. Agent antimicrobien comprenant le composé tréhalose défini par la revendication 1 en tant que principe actif.
